# EUROPEAN PATENT SPECIFICATION

(11) **EP 4 511 108 B1**
(45) Date of publication and mention of the grant of the patent: **13.05.2026**
(21) Application number: 23736808.9
(22) Date of filing: 06.04.2023
(51) Int. Cl.: A61B 5/02, G16H 40/67, G16H 50/20, A61B 5/00, G16H 50/30, A61B 5/145

(54) **BIOCHEMICAL SENSING FOR HEART FAILURE MANAGEMENT**
BIOCHEMISCHE MESSUNG ZUR VERWALTUNG VON HERZINSUFFIZIENZ
DÉTECTION BIOCHIMIQUE POUR GESTION D'INSUFFISANCE CARDIAQUE

(30) Priority: 22.04.2022 US 202263363456 P
(43) Date of publication of application: 26.02.2025
(73) Proprietor: Medtronic, Inc., Minneapolis, Minnesota 55432 (US)
(72) Inventor: STIGEN, Tyler, Minneapolis, Minnesota 55432 (US); O'BRIEN, Richard J., Minneapolis, Minnesota 55432 (US); TAYLOR, Amanda D., Minneapolis, Minnesota 55432 (US); PATEL, Nirav A., Minneapolis, Minnesota 55432 (US); YOON, Hyun J., Minneapolis, Minnesota 55432 (US); EISELE III, Val D, Minneapolis, Minnesota 55432 (US); WYSZYNSKI, Ryan D., Minneapolis, Minnesota 55432 (US); KRAUSE, Paul G., Minneapolis, Minnesota 55432 (US); SARKAR, Shantanu, Minneapolis, Minnesota 55432 (US); NELSON, Steven G., Minneapolis, Minnesota 55432 (US)
(74) Representative: Zimmermann & Partner Patentanwälte mbB
(86) International application number: PCT/IB2023/053531
(87) International publication number: WO 2023/203420

(56) References cited:
- US-A1- 2013 116 578
- US-A1- 2016 206 250
- US-A1- 2021 093 254

## Description

### TECHNICAL FIELD

The disclosure relates generally to medical devices and, more particularly, medical devices configured to monitor patient parameters.

### BACKGROUND

Some types of medical devices may be used to monitor one or more physiological parameters of a patient. Such medical devices may include, or may be part of a system that includes, sensors that detect signals associated with such physiological parameters. Values determined based on such signals may be used to assist in detecting changes in patient conditions, in evaluating the efficacy of a therapy, or in generally evaluating patient health. US 2021/0093254 A1 relates to determining likelihood of an adverse health event based on various physiological diagnostic states.

### SUMMARY

The claimed subject matter is defined in claim 1. Acute decompensated heart failure is a manifestation of worsening of heart failure (or broadly chronic illness) symptoms that may require heart failure hospital admission to relieve such patients of congestion and/or shortness of breath symptoms. A system configured for continuous (e.g., on a periodic or triggered basis without human intervention) ambulatory heart failure monitoring may be used to potentially predict worsening heart failure prior to development of severe symptoms requiring heart failure hospitalizations and provide clinicians with an opportunity to proactively treat the patient to avoid the heart failure hospital admission. Remote monitoring of some physiological parameters, such as thoracic impedance, e.g., via OptiVol^{™} available from Medtronic, Inc. of Minneapolis, Minnesota, or pulmonary artery pressure, is available. However, clinicians are often reluctant to make treatment decisions in the absence of information provided by all or a portion of a Basic Metabolic Panel (BMP) (e.g., sodium, chloride, potassium, bicarbonate, BUN, creatinine, glucose, etc.) or an International Normalized Ration (INR), a measure of clotting time, or in the absence of information regarding an infection status of the patient. Such measures provide a more complete picture of a patient's health status. Biochemical parameters, such as those of the BMP, are usually measured by a blood draw and a laboratory analysis, a finger stick point of care test, or by short-term (measured in days) percutaneous sensors.

In general, this disclosure is directed to techniques for using a medical device system to monitor physiological parameters of a patient, such as a heart failure patient. Sensors may be located on one or more implantable medical devices (IMDs), wearable devices, point of care finger stick devices, and/or other external devices, for sensing physiological parameters of a patient. Processing circuitry of the medical device system may execute an algorithm (e.g., TriageHF^{™} available from Medtronic, Inc. or a similar algorithm) to determine a likelihood of an impending heart failure decompensation event based on these sensed physiological parameters. As used herein "impending" may mean within less than 30 days, and in some examples within 7-10 days.

Many sensors for sensing physiological parameters relevant to heart failure have much longer lifespans than biochemical sensors which may be used to determine biochemical parameters, such as those of a BMP, or bacterial sensors which may be used to determine bacterial parameters. Because such biochemical and bacterial sensors may have a much shorter lifespan than other sensors and because clinicians are reluctant to hospitalize a heart failure patient without a recent BMP or a known bacterial infection, it may be desirable to deploy such sensors when an impending heart failure decompensation event is detected. Data sensed by such sensors may be further input into the algorithm to increase the accuracy of the initial assessment of the likelihood that there may be an impending heart failure decompensation event.

According to the techniques of this disclosure, a machine learning model or other algorithm configured to determine a likelihood of an impending heart failure decompensation event may be implemented in an implantable medical device (IMD), in a wearable device, such as a smart watch, in a smart phone that the patient may carry with them, on one or more servers, in a cloud computing environment, or the like. The device may execute the algorithm and may generate an instruction for output to a patient, caregiver or healthcare monitoring system, when the device determines that the likelihood of an impending heart failure decompensation event, based on sensed physiological parameters, meets a threshold. This instruction may automatically prescribe for the patient or caregiver to conduct a fingerstick measure, insert a percutaneous sensor, or insert an implantable sensor. Alternatively, or additionally, if the IMD includes one or more biochemical sensors or one or more bacterial sensors on or near the surface of the IMD, the device executing the algorithm could activate one or more biochemical sensors or one or more bacterial sensors, based on determining that the likelihood of an impending heart failure decompensation event meets a threshold. Such sensed biochemical parameters and/or bacterial parameters may be utilized by the device executing the algorithm to provide further precision in a determination of whether the patient should seek admission to a hospital or emergency room and/or whether a change in treatment is desirable.

Additionally, in the absence of a trigger from an indication of an impending heart failure decompensation, any one of these techniques for biochemical sensing or bacterial sensing, or a combination of these techniques, may be used to establish a baseline measure and trends over time. The frequency of such measurements could be predetermined, programmed and/or remotely activated by a clinician, be based on the patient's pattern of change in sensed biochemical parameters, bacterial parameters, and/or physiological parameters over time, or be determined by a machine learning model. A clinician may use such baseline measure and trends over time to monitor the cardiac health or comorbidities of the patient which may facilitate earlier detection of medical conditions requiring treatment, or to guide selection of medications, dosages or the like when treating the patient.

Because physicians are often reluctant to make treatment decisions in the absence of information provided by BMP or INR or in the absence of information regarding an infection status of a patient, it may be desirable to provide for the remote monitoring of such parameters. Because biochemical and/or bacterial sensors may have a shorter lifespan when in use than other types of sensors which may be used to sense physiological parameters of a patient, such as sensors of an implantable medical device, it may be desirable only to deploy biochemical and/or bacterial sensors when any physiological parameters that may be monitored, indicate that there is a likely impending heart failure decompensation event of the patient. Additionally, it may be desirable to deploy the biochemical and/or bacterial sensor(s) for a patient remotely from their clinician so that the patient does not need to make an office visit to deploy such sensor(s). Data collected by the biochemical and/or bacterial sensor(s) may be used to improve or verify the data collected by other sensors, so as to better predict an impending heart failure decompensation event, improve patient outcomes, and increase hospital efficiency. The techniques of this disclosure may address these issues by providing for at least one of generating an instruction for output to a user to deploy at least one of one or more biochemical sensors or one or more bacterial sensors, or controlling an implantable medical device to automatically deploy at least one of one or more biochemical sensors or one or more bacterial sensors when a likelihood that a heart failure decompensation event, determined based on other sensor data, meets a threshold.

Systems including one or more IMDs that incorporate such sensors and may be implanted in a patient over months or years and perform numerous operations per second on the data to enable the systems herein to determine varying risk levels of heart failure decompensation. Using techniques of this disclosure with an IMD may be advantageous when a physician cannot be continuously present with the patient over weeks or months to evaluate the physiological parameters and/or where performing the operations on the data described herein (e.g., application of a machine learning model) could not practically be performed in the mind of a physician. Using the techniques of this disclosure with autonomously/continuously operating IMDs and computing devices may provide a clinical advantage in timely detecting changes in a patient's condition and providing timely alerts to the patient and/or caregiver.

In some examples, the techniques and systems of this disclosure may use a machine learning model, e.g., a Bayesian probability model, to more accurately determine a risk level of a heart failure event based on physiological data collected by an IMD. In some examples, the machine learning model is trained with a set of training instances, where one or more of the training instances comprise data that indicate relationships between various sets of input data and risk level outputs, e.g., heart failure event likelihoods or probabilities. Because the machine learning model is trained with potentially thousands or millions of training instances, the machine learning model may reduce the amount of error in determining heart failure event risk. Reducing errors using the techniques of this disclosure may provide one or more technical and clinical advantages, such as increasing reliability of another device, user, and/or clinician on the accuracy of determining a patient's condition and improve resulting treatment of the patient and patient outcomes.

In some examples, a medical device system includes: memory configured to store first sensor data; and processing circuitry communicatively coupled to the memory, the processing circuitry being configured to: receive the first sensor data; determine, based on the first sensor data, a likelihood that a heart failure decompensation event of a patient is impending; compare the likelihood to a first threshold; and based on the likelihood satisfying the first threshold, at least one of a) generate a first instruction for output to a user to deploy at least one of one or more biochemical sensors or one or more bacterial sensors, or b) control an implantable medical device to automatically deploy at least one of one or more biochemical sensors or one or more bacterial sensors.

In some examples, a method includes receiving, by processing circuitry, first sensor data; determining, by the processing circuitry and based on the first sensor data, a likelihood that a heart failure decompensation event of a patient is impending; comparing, by the processing circuitry, the likelihood to a first threshold; based on the likelihood satisfying the first threshold, at least one of a) generating, by the processing circuitry, a first instruction for output to a user to deploy at least one of one or more biochemical sensors or one or more bacterial sensors, or b) controlling an implantable medical device, by the processing circuitry, to deploy at least one of one or more biochemical sensors or one or more bacterial sensors.

In some examples, a non-transitory computer-readable medium includes instructions, which when executed, cause processing circuitry to: receive first sensor data; determine, based on the first sensor data, a likelihood that a heart failure decompensation event of a patient is impending; compare the likelihood to a first threshold; based on the likelihood satisfying the first threshold, at least one of a) generate a first instruction for output to a user to deploy at least one of one or more biochemical sensors or one or more bacterial sensors, or b) control an implantable medical device to automatically deploy at least one of one or more biochemical sensors or one or more bacterial sensors.

This summary is intended to provide an overview of the subject matter described in this disclosure. It is not intended to provide an exclusive or exhaustive explanation of the systems, device, and methods described in detail within the accompanying drawings and description below. Further details of one or more examples of this disclosure are set forth in the accompanying drawings and in the description below. Other features, objects, and advantages will be apparent from the description and drawings, and from the claims.

### BRIEF DESCRIPTION OF DRAWINGS

FIG. 1 illustrates the environment of an example medical device system in conjunction with a patient, in accordance with one or more techniques of this disclosure.
FIG. 2 is a conceptual drawing illustrating an example configuration of the implantable medical device (IMD) of the medical device system of FIG. 1, in accordance with one or more techniques described herein.
FIG. 3 is a functional block diagram illustrating an example configuration of the IMD of FIGS. 1 and 2, in accordance with one or more techniques described herein.
FIGS. 4A and 4B are block diagrams illustrating two additional example IMDs that may be substantially similar to the IMD of FIGS. 1-3, but which may include one or more additional features, in accordance with one or more techniques described herein.
FIG. 5 is a block diagram illustrating an example configuration of components of the external device of FIG. 1, in accordance with one or more techniques of this disclosure.
FIG. 6 is a conceptual diagram illustrating an example sensor system which may be deployed by a patient or caregiver according to the techniques of this disclosure.
FIG. 7 is a conceptual diagram illustrating another example sensor system which may be deployed by a patient or caregiver according to the techniques of this disclosure.
FIG. 8 is a block diagram illustrating an example system that includes an access point, a network, external computing devices, such as a server, and one or more other computing devices, which may be coupled to the IMD of FIGS. 1-4, an external device, and processing circuitry via a network, in accordance with one or more techniques described herein.
FIG. 9 is a flow diagram illustrating an example of techniques to determine when to deploy biochemical sensors or bacterial sensors according to one or more aspects of this disclosure.
FIG. 10 is a conceptual diagram illustrating an example machine learning model configured to determine an extent to which data of a patient indicates a heart failure event.
FIG. 11 is a conceptual diagram illustrating an example training process for an artificial intelligence model, in accordance with examples of the current disclosure.

Like reference characters denote like elements throughout the description and figures.

### DETAILED DESCRIPTION

This disclosure describes techniques for determining when to deploy biochemical sensors or bacterial sensors in a heart failure or other patient. Data from such sensors may be used to improve the accuracy of a determined likelihood that heart failure decompensation event is impending. By improving the accuracy of the determined likelihood that a heart failure decompensation event is impending, the techniques of this disclosure may improve hospital resource usage, and may lead to early, more effective treatment. Data from such sensors may also be used to establish baseline biochemical and/or bacterial parameters for a patient and to track the patient health over time.

The techniques of this disclosure may facilitate the remote, ambulatory, monitoring and management of a heart failure patient, peripheral vascular disease patient, diabetes patient, patient at risk of deep vein thrombosis, and/or other cardiac patient. By providing for the remote and ambulatory monitoring of potential impending heart failure decompensation events, including improving the accuracy thereof by using data from deployed biochemical and/or bacterial sensors, the techniques of this disclosure may facilitate early intervention by a clinician during deteriorating conditions of the patient, faster detection of actionable events, reduced hospitalization, better management of medications, such as heart failure medications, which may improve patient outcomes and patient quality of life.

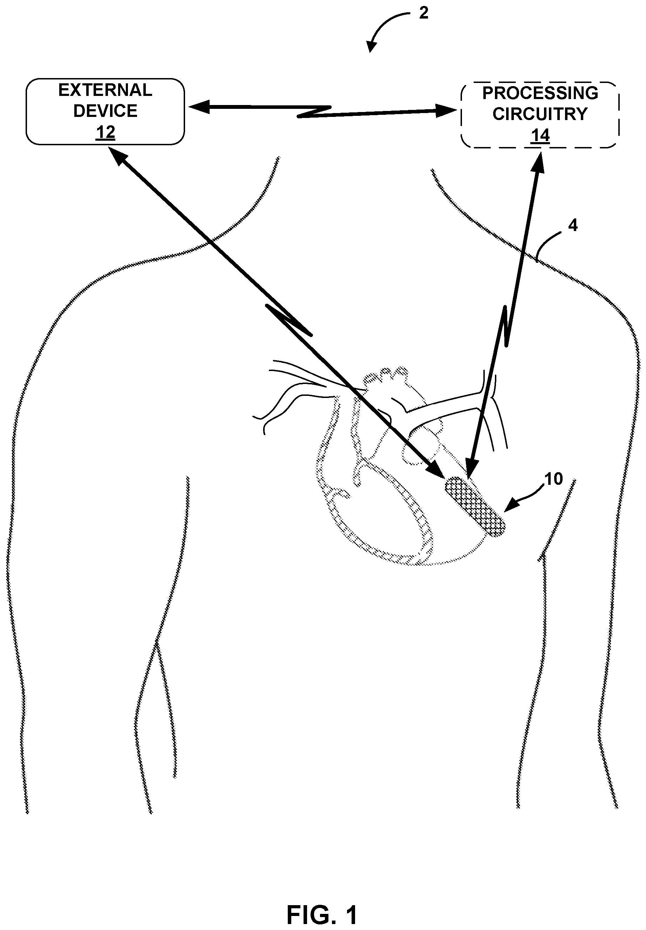

FIG. 1 illustrates the environment of an example medical device system 2 in conjunction with a patient 4, in accordance with one or more techniques of this disclosure. While the techniques described herein are generally described in the context of an insertable cardiac monitor and/or an external device, the techniques of this disclosure may be implemented in any implantable medical device or external device or combination thereof, such as a pacemaker, a defibrillator, a cardiac resynchronization therapy device, an implantable pulse generator, an intra-cardiac pressure measuring device, a ventricular assist device, a pulmonary artery pressure device, a subcutaneous blood pressure device, or an external device having a telemetry system and weight sensors, blood pressure sensors, pulse oximeters, activity sensors, patch systems, implantable biochemical sensor or bacterial sensors, implantable bacterial sensors, percutaneous biochemical sensor or bacterial sensors, percutaneous bacterial sensors, wearable biochemical sensor or bacterial sensors, wearable bacterial sensors, point of care finger stick sensors, or the like, configured to sense physiological parameters of a patient. The example techniques may be used with an IMD 10, which may be in wireless communication with at least one of external device 12 and other devices not pictured in FIG. 1. Processing circuitry 14 is conceptually illustrated in FIG. 1 as separate from IMD 10 and external device 12, but may be processing circuitry of IMD 10 and/or processing circuitry of external device 12. In general, the techniques of this disclosure may be performed by processing circuitry 14 of one or more devices of a system, such as one or more devices that include sensors that provide signals, or processing circuitry of one or more devices that do not include sensors, but nevertheless analyze signals using the techniques described herein. For example, another external device (not pictured in FIG. 1) may include at least a portion of processing circuitry 14, the other external device configured for remote communication with IMD 10 and/or external device 12 via a network.

In some examples, IMD 10 is implanted outside of a thoracic cavity of patient 4 (e.g., subcutaneously in the pectoral location illustrated in FIG. 1). IMD 10 may be positioned near the sternum near or just below the level of patient 4's heart, e.g., at least partially within the cardiac silhouette. For other medical conditions, IMD 10 may be implanted in other appropriate locations, such as the interstitial space, abdomen, back of arm, wrist, etc. In some examples, IMD 10 takes the form of a Reveal LINQ^{™} or LINQ II^{™} Insertable Cardiac Monitor (ICM), available from Medtronic, Inc.

Clinicians sometimes diagnose patients with medical conditions based on one or more observed physiological signals collected by physiological sensors, such as electrodes, optical sensors, chemical sensors, temperature sensors, acoustic sensors, and motion sensors. In some cases, clinicians apply non-invasive sensors to patients in order to sense one or more physiological signals while a patient is in a clinic for a medical appointment. However, in some examples, physiological markers (e.g., arrythmia, etc.) of a patient condition are rare or are difficult to observe over a relatively short period of time. As such, in these examples, a clinician may be unable to observe the physiological markers needed to diagnose a patient with a medical condition or effectively treat the patient while monitoring one or more physiological signals of the patient during a medical appointment. Moreover, an impending heart failure decomposition event may be difficult to diagnose during a medical appointment. In the example illustrated in FIG. 1, IMD 10 is implanted within patient 4 to continuously record one or more physiological signals, such as an electrocardiogram (ECG), electromyogram (EMG), impedance, respiration, activity, posture, blood oxygen saturation, or other physiological signals, of patient 4 over an extended period of time.

In some examples, IMD 10 includes a plurality of electrodes. The plurality of electrodes is configured to detect signals that enable processing circuitry 14, e.g., of IMD 10, to monitor and/or record physiological parameters of patient 4. For example, the plurality of electrodes may be configured to sense an ECG, EMG, impedance, or the like, of patient 4. IMD 10 may additionally or alternatively include one or more optical sensors, accelerometers, temperature sensors, chemical sensors, light sensors, pressure sensors, in some examples. Such sensors may detect one or more physiological parameters indicative of a patient condition. The physiological parameters may include impedance, such as interstitial or other impedance indicative of fluid accumulation analyzed according to the OptiVol^{™} algorithm or another algorithm that determines a fluid index based on impedance, patient activity, anti-tachycardia/anti-fibrillation burden, ventricular rate during anti-tachycardia/anti-fibrillation, percentage of ventricular pacing, shocks, treated ventricular tachycardia/ventricular fibrillation, night ventricular rate, heart rate variability, heart sounds, lung sounds, and/or other physiological parameters. In some examples, additional sensors may be located on other devices (not shown in FIG. 1) which may also sense physiological parameters of patient 4.

Sensor data may be collected by various devices such as implantable therapy devices, implantable monitoring devices, wearable devices, point of care devices, and noncontact sensors in the home or vehicle or other area frequented by the patient or a combination of such sensor platforms. The sensor data collected may be relevant to the disease state (e.g., heart failure) or comorbidities (e.g., chronic obstructive pulmonary disease (COPD), kidney disease, etc.) or for diagnosing a suspected comorbidity. For patients with multiple comorbidities, it may be possible to perform a differential diagnosis between different sources of a problem, e.g., heart failure decompensation, COPD exacerbation, pneumonia, etc. This would permit a clinician to prescribe appropriate therapies to address the patient's condition, such as diuretics, antibiotics, encourage fluids, etc.

Processing circuitry 14 may process the sensed physiological parameters (whether sensed by IMD 10, another device(s), or any combination thereof). For example, processing circuitry 14 may execute an algorithm (such as TriageHF^{™} or a similar algorithm) configured to determine a likelihood of an impending heart failure decompensation event based on sensed physiological parameters of patient 4.

This likelihood may be a percentage chance that patient 4 may have an impending heart failure decompensation event, a rating (e.g., high, medium, low), a score (e.g., on a scale of 1-10) or the like. Processing circuitry 14 may monitor such sensed physiological parameters as impedance (e.g., using a fluid index algorithm), patient activity, atrial tachycardia (AT)/atrial fibrillation (AF) burden, ventricular rate during AT/AF, percentage of ventricular pacing, shocks, treated ventricular tachycardia/ventricular fibrillation, night ventricular rate, heart rate variability, heart sounds, lung sounds, and/or other physiological parameters. In some examples, processing circuitry 14 may compare each of the sensed physiological parameters used by the algorithm to parameter-specific thresholds and processing circuitry 14 may apply a Bayesian probabilistic model (e.g., a Bayesian Belief Network) to results of the comparisons to determine a likelihood that patient 4 may have an impending heart failure decompensation event. Further details on how to determine a likelihood that a heart failure decomposition event is impending may be found in commonly-assigned U.S. Patent Publication 2020-0187864 A1 published on June 18, 2020, and U.S. Patent Publication 2021-0093254 A1, the entire contents of which are hereby incorporated by reference.

For example, processing circuitry 14 may derive features (such as a fluid index) from aggregated data. Raw data, aggregated data, and determined physiological features may be combined in the cloud using machine learning algorithms or deep learning models to generate clinically actionable insights. In some examples, the sensed physiological parameters may be collected and processed to provide an index or score of patient 4's health. There are several examples of such scoring systems that are in clinical use today. One example is the APACHE (Acute Physiology and Chronic Health Evaluation) IV scoring system. In some examples, processing circuitry 14 may continuously calculate such a score, or a similar score. Processing circuitry 14 may attempt to determine any source of changes in patient 4's health based on such sensed physiological parameters and, when appropriate, send an indication to a clinician regarding the change in the score and the source of the changes in the patient's health status with suggested treatment options. Processing circuitry 14 may also provide to patient 4 or a caregiver of patient 4 any prescription changes in medications or directions to use a biochemical sensor or bacterial sensor. These scores and directions could be provided via external device 12. Such a biochemical sensor may include a multianalyte sensor that is configured to sense BMP parameters, a multianalyte sensor that is configured to sense other biochemical parameters, a multianalyte sensor that is configured to sense some BMP parameters, a multianalyte sensor that is configured to sense some BMP parameters and some other biochemical parameters, or a single analyte sensor. Such a bacterial sensor may include an antigen sensor configured to sense an antigen present on a membrane of a bacterium, a byproduct sensor configured to sense a biproduct particular to a bacterium, a sensor configured to sense something that normally is not present in a human body, or the like.

Processing circuitry 14 may receive sensor data. For example, processing circuitry 14 may receive sensor data from IMD 10 or other sensors in or about patient 4. Processing circuitry may determine, based on the sensor data, a likelihood that a heart failure decompensation event is impending. Processing circuitry 14 may compare the likelihood to a first threshold. For example, if the likelihood meets the first threshold it may indicate that a heart failure decompensation event is likely to occur within the next 30 days. Based on the likelihood satisfying the first threshold, processing circuitry 14 may generate an instruction for output to deploy one or more biochemical or bacterial sensors, for example, via external device 12 to patient 4 or a caregiver of patient 4. In some examples, processing circuitry 14 may also generate an indication for output for review by a clinician, e.g., via external device 12. Such an indication may include a heart failure score, sensed physiological parameters, or the like. In some examples, processing circuitry 14 may generate further instructions or messages for patient 4 or a caregiver regarding when patient 4 or the caregiver should replace the biochemical sensor or bacterial sensor and/or when it is no longer necessary to replace the biochemical sensor or bacterial sensor.

In some examples, IMD 10 may include one or more biochemical sensors and/or one or more bacterial sensors which may be disposed on a surface or near the surface of IMD 10. Such sensors may be covered by welds, doors, or the like to keep the biochemical sensor(s) and/or bacterial sensor(s) inactive to preserve their life. In such a case, processing circuitry 14 may control IMD 10 to deploy one or more biochemical sensors or bacterial sensors based on the likelihood that a heart failure decompensation event of patient 4 is impending meeting the threshold. This may be performed alone or together with generating the instructions for output to patient 4 or a caregiver of patient 4 as described above.

For example, processing circuitry 14 may control IMD 10 to release, perforate, dissolve, or otherwise open the weld or door of one or more of the biochemical sensors or bacterial sensors. In some examples, the door or weld may include a thin gold foil which IMD 10 may electro-perforate or electro-vaporize to deploy a given biochemical or bacterial sensor.

In some examples, regardless of the determined likelihood of an impending heart failure decompensation event meeting the threshold, a clinician may desire to periodically monitor biochemical parameters and/or bacterial parameters of patient 4, for example to determine a baseline or monitor trends over time in the cardiac health of patient 4. In such cases, the clinician via external device 12 may provide patient 4 or a caregiver of patient 4 with instructions to deploy one or more biochemical sensor or bacterial sensors. Additionally, or alternatively, the clinician may trigger the opening of the weld or door of one or more biochemical sensor or bacterial sensors of IMD 10. This triggering may be performed by programming IMD 10, via external device 12, to open the weld or door of the one or more biochemical sensor or bacterial sensors at a programed time, at a programmed time interval, or in real-time based on a command sent be the clinician via external device 12. In some examples, the times with which IMD 10 may open a weld or door or with which external device 12 provides patient 4 or a caregiver of patient 4 with instructions to deploy one or more biochemical sensor or bacterial sensors may be predetermined.

In some examples, IMD 10, external device 12, and/or processing circuitry 14 may apply a machine learning model to determine when to trigger the deployment, either by patient 4 or a caregiver, or by IMD 10, of one or more biochemical sensors and/or one or more bacterial sensors. The machine learning model may be trained, for example, on particular physiological parameters, biochemical parameters, bacterial parameters, disease states, comorbidities, activities, postures, family history, age, or the like of patient 4, or of a population of a plurality of patients.

Potential machine learning models may be configured as Bayesian algorithms, clustering algorithms, decision-tree algorithms, regularization algorithms, regression algorithms (e.g., linear or logistic), instance-based algorithms, artificial neural network algorithms, deep learning algorithms, random forest algorithms, support vector machines, dimensionality reduction algorithms and the like. Various examples of specific algorithms include Naive Bayes, Bayesian Linear Regression, Boosted Decision Tree Regression, and Neural Network Regression, Back Propagation Neural Networks, Convolution Neural Networks (CNN), Long Short Term Networks (LSTM), the Apriori algorithm, K-Means Clustering, k-Nearest Neighbour (kNN), Learning Vector Quantization (LVQ), Self-Organizing Map (SOM), Locally Weighted Learning (LWL), Ridge Regression, Least Absolute Shrinkage and Selection Operator (LASSO), Elastic Net, and Least-Angle Regression (LARS), Principal Component Analysis (PCA) and Principal Component Regression (PCR).

For example, a k-means clustering model may be used having two clusters: one for deploying the one or more biochemical sensor and one for not deploying the one or more biochemical sensor. Each time the model is run may be associated with a vector that includes variables for, e.g., particular physiological parameters, biochemical parameters, disease states, comorbidities, activities, postures, family history, age, of patient 4, how many biochemical sensors remain on IMD 10 or within the possession of patient 4 or a caregiver, or the like. The location of the vector in a given one of the clusters may be indicative of whether IMD 10, patient 4 or a caregiver should deploy one or more biochemical sensors. For example, if the vector falls within the deploy the one or more biometric sensor cluster, IMD 10 may deploy the one or more biochemical sensor and/or external device 12 may inform patient 4 or a caregiver to deploy one or more biochemical sensors. In some examples, a more complicated model may be used with additional clusters for different types of biochemical sensors, such that the deployment of different types of biochemical sensors may be determined through the machine learning model. A similar model may be used to determine when to deploy one or more bacterial sensor.

External device 12 may be a hand-held computing device with a display viewable by the user and an interface for providing input to external device 12 (i.e., a user input mechanism). For example, external device 12 may include a small display screen (e.g., a liquid crystal display (LCD) or a light emitting diode (LED) display) that presents information to the user, such as instructions to deploy one or more biochemical sensor or bacterial sensors. In addition, external device 12 may include a touch screen display, keypad, buttons, a peripheral pointing device, voice activation, or another input mechanism that allows the user to navigate through the user interface of external device 12 and provide input. If external device 12 includes buttons and a keypad, the buttons may be dedicated to performing a certain function, e.g., a power button, the buttons and the keypad may be soft keys that change in function depending upon the section of the user interface currently viewed by the user, or any combination thereof.

In other examples, external device 12 may be a larger workstation or a separate application within another multi-function device, rather than a dedicated computing device. For example, the multi-function device may be a notebook computer, tablet computer, workstation, one or more servers, cellular phone, personal digital assistant, or another computing device that may run an application that enables the computing device to operate as a secure device.

When external device 12 is configured for use by the clinician, external device 12 may be used to transmit instructions to IMD 10 and to receive measurements, such sensed physiological parameters, sensed biochemical parameters, sensed bacterial parameters, heart failure scores, indications of the determined likelihood of an impending heart failure decompensation event, or the like. Example instructions may include requests to set electrode combinations for sensing and any other information that may be useful for programming into IMD 10, such as when to deploy one or more biochemical sensors and/or one or more bacterial sensors. The clinician may also configure and store operational parameters for IMD 10 within IMD 10 with the aid of external device 12. In some examples, external device 12 assists the clinician in the configuration of IMD 10 by providing a system for identifying potentially beneficial operational parameter values.

Whether external device 12 is configured for clinician or patient use, external device 12 is configured to communicate with IMD 10 and, optionally, another computing device (not illustrated in FIG. 1), via wireless communication. External device 12, for example, may communicate via near-field communication technologies (e.g., inductive coupling, NFC or other communication technologies operable at ranges less than 10-20 cm) and far-field communication technologies (e.g., RF telemetry according to the 802.11 or Bluetooth^{®} specification sets, or other communication technologies operable at ranges greater than near-field communication technologies).

Processing circuitry 14, in some examples, may include one or more processors that are configured to implement functionality and/or process instructions for execution within IMD 10 and/or external device 12. For example, processing circuitry 14 may be capable of processing instructions stored in a storage device. Processing circuitry 14 may include, for example, microprocessors, digital signal processors (DSPs), application specific integrated circuits (ASICs), field-programmable gate arrays (FPGAs), or equivalent discrete or integrated logic circuitry, or a combination of any of the foregoing devices or circuitry. Accordingly, processing circuitry 14 may include any suitable structure, whether in hardware, software, firmware, or any combination thereof, to perform the functions ascribed herein to processing circuitry 14.

Processing circuitry 14 may represent processing circuitry located within any combination of IMD 10 and/or external device 12. In some examples, processing circuitry 14 may be entirely located within a housing of IMD 10. In other examples, processing circuitry 14 may be entirely located within a housing of external device 12. In other examples, processing circuitry 14 may be located within any combination of IMD 10, external device 12, and another device or group of devices that are not illustrated in FIG. 1. As such, techniques and capabilities attributed herein to processing circuitry 14 may be attributed to any combination of IMD 10, external device 12, and other devices that are not illustrated in FIG. 1.

In some examples, IMD 10 includes one or more motion sensors, such as accelerometers. An accelerometer of IMD 10 may collect an accelerometer signal which reflects a measurement of a motion of patient 4. In some cases, the accelerometer may collect a three-axis accelerometer signal indicative of patient 4's movements within a three-dimensional Cartesian space. For example, the accelerometer signal may include a vertical axis accelerometer signal vector, a lateral axis accelerometer signal vector, and a frontal axis accelerometer signal vector. The vertical axis accelerometer signal vector may represent an acceleration of patient 4 along a vertical axis, the lateral axis accelerometer signal vector may represent an acceleration of patient 4 along a lateral axis, and the frontal axis accelerometer signal vector may represent an acceleration of patient 4 along a frontal axis. In some cases, the vertical axis substantially extends along a torso of patient 4 when patient 4 from a neck of patient 4 to a waist of patient 4, the lateral axis extends across a chest of patient 4 perpendicular to the vertical axis, and the frontal axis extends outward from and through the chest of patient 4, the frontal axis being perpendicular to the vertical axis and the lateral axis. In some examples, processing circuitry 14 may be configured to identify, based on one of more accelerometer signals, a posture or activity of patient 4. In some examples, the sensed physiological parameters by IMD 10 may include the posture and/or activity of patient 4. Such postures and/or activities of patient 4 may be used when determining the likelihood of an impending heart failure decompensation event.

Although in one example IMD 10 takes the form of an ICM, in other examples, IMD 10 takes the form of any one or more of an ICM, a pacemaker, a defibrillator, a cardiac resynchronization therapy device, an implantable pulse generator, an intra-cardiac pressure measuring device, a ventricular assist device, a pulmonary artery pressure device, a subcutaneous blood pressure device, or the like. The physiological parameters, the biochemical parameters, and/or the bacterial parameters may be sensed or determined using one or more of the aforementioned devices, as well as external devices such as external device 12.

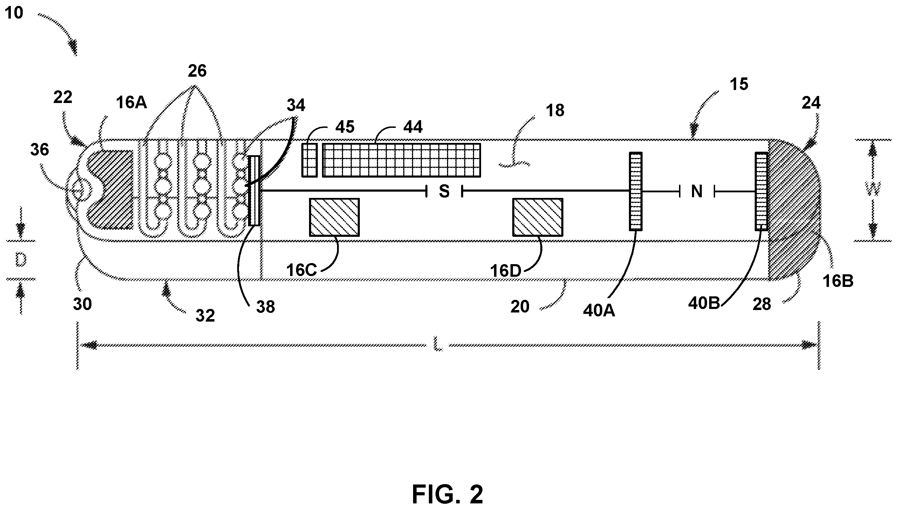

FIG. 2 is a conceptual drawing illustrating an example configuration of IMD 10 of the medical device system 2 of FIG. 1, in accordance with one or more techniques described herein. In the example shown in FIG. 2, IMD 10 may be a leadless, subcutaneously- or vascularly-implantable monitoring device having housing 15, proximal electrode 16A, and distal electrode 16B. Housing 15 may further include first major surface 18, second major surface 20, proximal end 22, and distal end 24. In some examples, IMD 10 may include one or more additional electrodes 16C, 16D positioned on one or both of major surfaces 18, 20 of IMD 10. Housing 15 encloses electronic circuitry located inside the IMD 10, and protects the circuitry contained therein from fluids such as body fluids (e.g., blood). In some examples, electrical feedthroughs provide electrical connection of electrodes 16A-16D, and antenna 26, to circuitry within housing 15. In some examples, electrode 16B may be formed from an uninsulated portion of conductive housing 15.

In the example shown in FIG. 2, IMD 10 is defined by a length L, a width W, and thickness or depth D. In this example, IMD 10 is in the form of an elongated rectangular prism in which length L is significantly greater than width W, and in which width W is greater than depth D. However, other configurations of IMD 10 are contemplated, such as those in which the relative proportions of length L, width W, and depth D vary from those described and shown in FIG. 2. In some examples, the geometry of the IMD 10, such as the width W being greater than the depth D, may be selected to allow IMD 10 to be inserted under the skin of the patient using a minimally invasive procedure and to remain in the desired orientation during insertion. In addition, IMD 10 may include radial asymmetries (e.g., the rectangular shape) along a longitudinal axis of IMD 10, which may help maintain the device in a desired orientation following implantation.

In some examples, a spacing between proximal electrode 16A and distal electrode 16B may range from about 30-55 mm, about 35-55 mm, or about 40-55 mm, or more generally from about 25-60 mm. Overall, IMD 10 may have a length L of about 20-30 mm, about 40-60 mm, or about 45-60 mm. In some examples, the width W of major surface 18 may range from about 3-10 mm, and may be any single width or range of widths between about 3-10 mm. In some examples, a depth D of IMD 10 may range from about 2-9 mm. In other examples, the depth D of IMD 10 may range from about 2-5 mm, and may be any single or range of depths from about 2-9 mm. In any such examples, IMD 10 is sufficiently compact to be implanted within the subcutaneous space of patient 4 in the region of a pectoral muscle.

IMD 10, according to an example of the present disclosure, may have a geometry and size designed for ease of implant and patient comfort. Examples of IMD 10 described in this disclosure may have a volume of 3 cubic centimeters (cm³) or less, 1.5 cm³ or less, or any volume therebetween. In addition, in the example shown in FIG. 2, proximal end 22 and distal end 24 are rounded to reduce discomfort and irritation to surrounding tissue once implanted under the skin of patient 4.

In the example shown in FIG. 2, first major surface 18 of IMD 10 faces outward towards the skin, when IMD 10 is inserted within patient 4, whereas second major surface 20 is faces inward toward musculature of patient 4. Thus, first and second major surfaces 18, 20 may face in directions along a sagittal axis of patient 4 (see FIG. 1), and this orientation may be generally maintained upon implantation due to the dimensions of IMD 10.

Proximal electrode 16A and distal electrode 16B may be used to sense cardiac EGM signals (e.g., ECG signals) when IMD 10 is implanted subcutaneously in patient 4. In some examples, processing circuitry of IMD 10 also may determine whether cardiac ECG signals of patient 4 are indicative of arrhythmia or other abnormalities, which processing circuitry of IMD 10 may evaluate in determining whether a medical condition (e.g., heart failure, sleep apnea, or COPD) of patient 4 has changed. The cardiac ECG signals may be stored in a memory of IMD 10, and data derived from the cardiac ECG signals and/or other sensor signals, such as an impending heart failure decompensation event may be transmitted via integrated antenna 26 to another device, such as external device 12. Additionally, in some examples, electrodes 16A, 16B may be used by communication circuitry of IMD 10 for tissue conductance communication (TCC) communication with external device 12 or another device.

In the example shown in FIG. 2, proximal electrode 16A is in close proximity to proximal end 22, and distal electrode 16B is in close proximity to distal end 24 of IMD 10. In this example, distal electrode 16B is not limited to a flattened, outward facing surface, but may extend from first major surface 18, around rounded edges 28 or end surface 30, and onto the second major surface 20 in a three-dimensional curved configuration. As illustrated, proximal electrode 16A is located on first major surface 18 and is substantially flat and outward facing. However, in other examples not shown here, proximal electrode 16A and distal electrode 16B both may be configured like proximal electrode 16A shown in FIG. 2, or both may be configured like distal electrode 16B shown in FIG. 2. In some examples, additional electrodes 16C and 16D may be positioned on one or both of first major surface 18 and second major surface 20, such that a total of four electrodes are included on IMD 10. Any of electrodes 16A-16D may be formed of a biocompatible conductive material. For example, any of electrodes 16A-16D may be formed from any of stainless steel, titanium, platinum, iridium, or alloys thereof. In addition, electrodes of IMD 10 may be coated with a material such as titanium nitride or fractal titanium nitride, although other suitable materials and coatings for such electrodes may be used.

In the example shown in FIG. 2, proximal end 22 of IMD 10 includes header assembly 32 having one or more of proximal electrode 16A, integrated antenna 26, anti-migration projections 34, and suture hole 36. Integrated antenna 26 is located on the same major surface (e.g., first major surface 18) as proximal electrode 16A, and may be an integral part of header assembly 32. In other examples, integrated antenna 26 may be formed on the major surface opposite from proximal electrode 16A, or, in still other examples, may be incorporated within housing 15 of IMD 10. Antenna 26 may be configured to transmit or receive electromagnetic signals for communication. For example, antenna 26 may be configured to transmit to or receive signals from a programmer via inductive coupling, electromagnetic coupling, tissue conductance, Near Field Communication (NFC), Radio Frequency Identification (RFID), Bluetooth^{®}, WiFi^{®}, or other proprietary or non-proprietary wireless telemetry communication schemes. Antenna 26 may be coupled to communication circuitry of IMD 10, which may drive antenna 26 to transmit signals to external device 12, and may transmit signals received from external device 12 to processing circuitry of IMD 10 via communication circuitry.

In some examples, IMD 10 may include several features for retaining IMD 10 in position once subcutaneously implanted in patient 4, so as to decrease the chance that IMD 10 migrates in the body of patient 4. For example, as shown in FIG. 2, housing 15 may include anti-migration projections 34 positioned adjacent integrated antenna 26. Anti-migration projections 34 may include a plurality of bumps or protrusions extending away from first major surface 18, and may help prevent longitudinal movement of IMD 10 after implantation in patient 4. In other examples, anti-migration projections 34 may be located on the opposite major surface as proximal electrode 16A and/or integrated antenna 26. In addition, in the example shown in FIG. 2 header assembly 32 includes suture hole 36, which provides another means of securing IMD 10 to the patient to prevent movement following insertion. In the example shown, suture hole 36 is located adjacent to proximal electrode 16A. In some examples, header assembly 32 may include a molded header assembly made from a polymeric or plastic material, which may be integrated or separable from the main portion of IMD 10.

In the example of FIG. 2, IMD 10 includes sensors 44. Sensors 44 may include one or more biochemical sensor and/or one or more bacterial sensors. Such biochemical sensor or bacterial sensors may be arranged in an array. Each biochemical sensor or bacterial sensor may be covered by a weld or door (not shown in FIG. 2) which may shield each biochemical sensor or bacterial sensor from exposure to the surrounding tissue of patient 4. When a particular biochemical sensor or bacterial sensor is to be deployed, IMD 10 may dissolve, perforate, or otherwise open the door to expose the biochemical sensor or bacterial sensor to the surrounding tissue of patient 4. In some examples, IMD 10 includes treatment 45. Treatment 45 may include one or more boluses of anti-biotic medication. Such boluses of anti-biotics may also each be covered by a weld or door which IMD 10 may dissolve, perforate, or otherwise open to release the bolus into patient 4. In some examples, IMD 10 may release a bolus of antibiotic medication based on a bacterial sensor signal indicating that there is an infection in patient 4.

In the example shown in FIG. 2, IMD 10 includes a light emitter 38, a proximal light detector 40A, and a distal light detector 40B positioned on housing 15 of IMD 10. Light detector 40A may be positioned at a distance S from light emitter 38, and a distal light detector 40B positioned at a distance S+N from light emitter 38. In other examples, IMD 10 may include only one of light detectors 40A, 40B, or may include additional light emitters and/or additional light detectors. Although light emitter 38 and light detectors 40A, 40B are described herein as being positioned on housing 15 of IMD 10, in other examples, one or more of light emitter 38 and light detectors 40A, 40B may be positioned, on a housing of another type of IMD within patient 4, such as a transvenous, subcutaneous, or extravascular pacemaker or ICD, or connected to such a device via a lead.

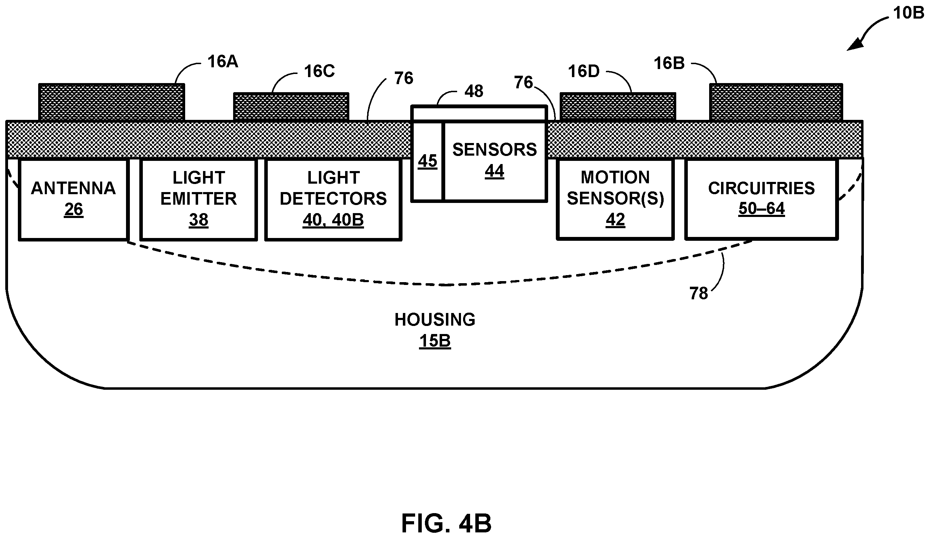

As shown in FIG. 2, light emitter 38 may be positioned on header assembly 32, although, in other examples, one or both of light detectors 40A, 40B may additionally or alternatively be positioned on header assembly 32. In some examples, light emitter 38 may be positioned on a medial section of IMD 10, such as part way between proximal end 22 and distal end 24. Although light emitter 38, light detectors 40A, 40B, and sensors 44 are illustrated as being positioned on first major surface 18, light emitter 38, light detectors 40A, 40B, and sensors 44 alternatively may be positioned on second major surface 20. In some examples, IMD may be implanted such that light emitter 38 and light detectors 40A, 40B face inward when IMD 10 is implanted, toward the muscle of patient 4, which may help minimize interference from background light coming from outside the body of patient 4. Light detectors 40A, 40B may include a glass or sapphire window, such as described below with respect to FIG. 4B, or may be positioned beneath a portion of housing 15 of IMD 10 that is made of glass or sapphire, or otherwise transparent or translucent.

In some examples, IMD 10 may include one or more additional sensors, such as one or more motion sensors (not shown in FIG. 2). Such motion sensors may be 3D accelerometers configured to generate signals indicative of one or more types of movement of the patient, such as gross body movement (e.g., motion) of the patient, patient posture, movements associated with the beating of the heart, or coughing, rales, or other respiration abnormalities, or the movement of IMD 10 within the body of patient 4. One or more of the parameters monitored by IMD 10 (e.g., bio impedance, EGM) may fluctuate in response to changes in one or more such types of movement. For example, changes in parameter values sometimes may be attributable to increased patient motion (e.g., exercise or other physical motion as compared to immobility) or to changes in patient posture, and not necessarily to changes in a medical condition. Thus, in some methods of identifying or tracking a medical condition of patient 4, it may be advantageous to account for such fluctuations when determining whether a change in a parameter is indicative of a change in a medical condition or when determining the likelihood of an impending heart failure decompensation event.

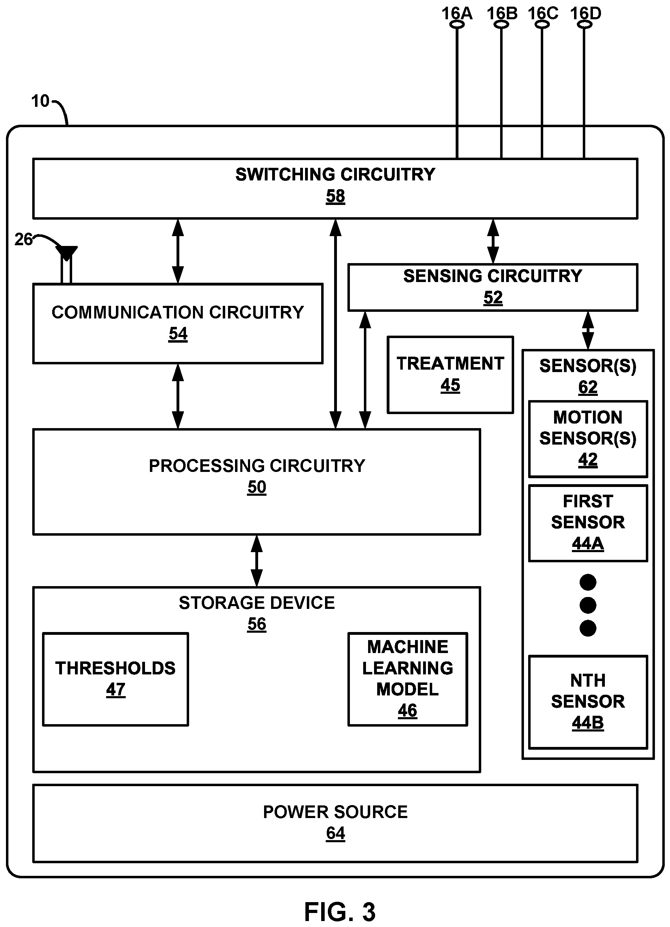

FIG. 3 is a functional block diagram illustrating an example configuration of IMD 10 of FIGS. 1 and 2, in accordance with one or more techniques described herein. In the illustrated example, IMD 10 includes electrodes 16, antenna 26, processing circuitry 50, sensing circuitry 52, communication circuitry 54, storage device 56, switching circuitry 58, sensors 62 including motion sensor(s) 42 (which may include an accelerometer) and a plurality of other sensors (e.g., first sensor 44A - Nth sensor 44N, collectively "sensors 44), treatment 45, and power source 64. Sensors 44 may include one or more biochemical sensor and/or one or more bacterial sensors which may be disposed on or near a surface of IMD 10. Each of biochemical sensors or bacterial sensors of sensors 44 may be covered by a weld or door so as to not expose the biochemical sensors or bacterial sensors to surrounding tissue of patient 4 until each biochemical sensor or bacterial sensor is deployed via dissolving, perforating, or otherwise opening the weld or door. In some examples, the one or more biochemical sensors and/or the one or more bacterial sensors may be arranged in an array.

Processing circuitry 50 may include fixed function circuitry and/or programmable processing circuitry. Processing circuitry 50 may include any one or more of a microprocessor, a controller, a DSP, an ASIC, an FPGA, or equivalent discrete or analog logic circuitry. In some examples, processing circuitry 50 may include multiple components, such as any combination of one or more microprocessors, one or more controllers, one or more DSPs, one or more ASICs, or one or more FPGAs, as well as other discrete or integrated logic circuitry. The functions attributed to processing circuitry 50 herein may be embodied as software, firmware, hardware or any combination thereof. In some examples, one or more techniques of this disclosure may be performed by processing circuitry 50.

Sensing circuitry 52 and communication circuitry 54 may be selectively coupled to electrodes 16A-16D via switching circuitry 58, as controlled by processing circuitry 50. Sensing circuitry 52 may monitor signals from electrodes 16A-16D in order to monitor electrical activity of heart (e.g., to produce an ECG). Sensing circuitry 52 also may monitor signals from sensors 62, which may include motion sensor(s) 42 (which may include an accelerometer) and sensors 44. In some examples, sensing circuitry 52 may include one or more filters and amplifiers for filtering and amplifying signals received from one or more of electrodes 16A-16D, motion sensor(s) 42, and/or sensors 44.

Communication circuitry 54 may include any suitable hardware, firmware, software or any combination thereof for communicating with another device, such as external device 12 or another IMD or sensor, such as a pressure sensing device. Under the control of processing circuitry 50, communication circuitry 54 may receive downlink telemetry from, as well as send uplink telemetry to, external device 12 or another device with the aid of an internal or external antenna, e.g., antenna 26. In addition, processing circuitry 50 may communicate with a networked computing device via an external device (e.g., external device 12) and a computer network, such as the Medtronic CareLink^{®} Network developed by Medtronic, Inc.

A clinician or other user may retrieve data from IMD 10 using external device 12, or by using another local or networked computing device configured to communicate with processing circuitry 50 via communication circuitry 54. The clinician may also program parameters of IMD 10 using external device 12 or another local or networked computing device.

In some examples, storage device 56 includes computer-readable instructions that, when executed by processing circuitry 50, cause IMD 10 and processing circuitry 50 to perform various functions attributed to IMD 10 and processing circuitry 50 herein. Storage device 56 may include any volatile, non-volatile, magnetic, optical, or electrical media, such as a random access memory (RAM), ferroelectric RAM (FRAM) read-only memory (ROM), non-volatile RAM (NVRAM), electrically-erasable programmable ROM (EEPROM), flash memory, or any other digital media.

Storage device 56 may also store machine learning model 46. In some examples, machine learning model 46 may be used by processing circuitry 50 to determine when to deploy one or more biochemical sensors and/or one or more bacterial sensors of sensors 44 and/or to instruct patient 4 or a caregiver to deploy one or more biochemical sensors and/or one or more bacterial sensors as discussed above. In some examples, processing circuitry 50 may employ machine learning model 46 or another machine learning model to determine a risk of a heart failure event based on data determined from sensors 44, and in some cases other values of other parameters, e.g., sensed via sensing circuitry 52 or sensors as described herein.

Power source 64 is configured to deliver operating power to the components of IMD 10. Power source 64 may include a battery and a power generation circuit to produce the operating power. In some examples, the battery is rechargeable to allow extended operation. In some examples, recharging is accomplished through proximal inductive interaction between an external charger and an inductive charging coil within external device 12. Power source 64 may include any one or more of a plurality of different battery types, such as nickel cadmium batteries and lithium ion batteries. A non-rechargeable battery may be selected to last for several years, while a rechargeable battery may be inductively charged from an external device, e.g., on a daily or weekly basis.

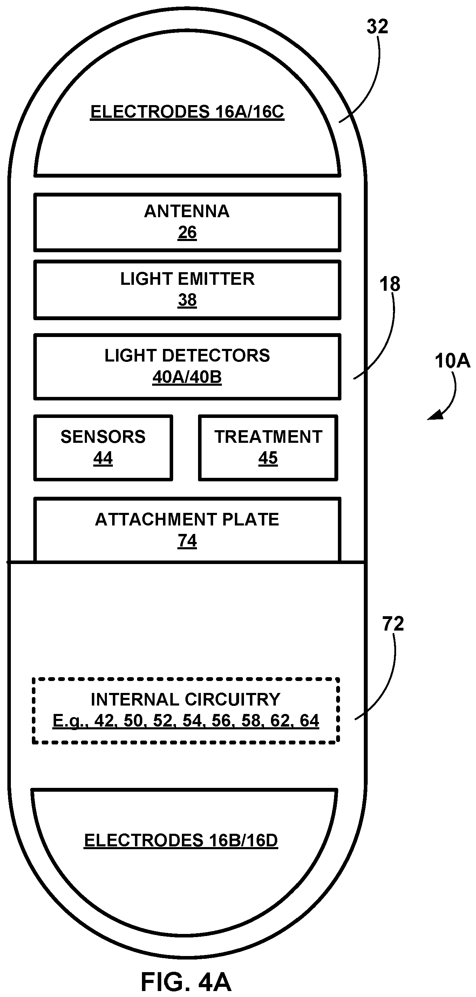

FIGS. 4A and 4B illustrate two additional example IMDs that may be substantially similar to IMD 10 of FIGS. 1-3, but which may include one or more additional features, in accordance with one or more techniques described herein. The components of FIGS. 4A and 4B may not necessarily be drawn to scale, but instead may be enlarged to show detail. FIG. 4A is a block diagram of a top view of an example configuration of an IMD 10A. FIG. 4B is a block diagram of a side view of example IMD 10B, which may include an insulative layer as described below.

FIG. 4A is a conceptual drawing illustrating another example IMD 10A that may be substantially similar to IMD 10 of FIG. 1. In addition to the components illustrated in FIGS. 1-3, the example of IMD 10 illustrated in FIG. 4A also may include a body portion 72, an attachment plate 74, and treatment 45. Attachment plate 74 may be configured to mechanically couple header assembly 32 to body portion 72 of IMD 10A. Body portion 72 of IMD 10A may be configured to house one or more of the internal components of IMD 10 illustrated in FIG. 3. such as one or more of processing circuitry 50, sensing circuitry 52, communication circuitry 54, storage device 56, switching circuitry 58, internal components of sensors 62, and power source 64. In some examples, body portion 72 may be formed of one or more of titanium, ceramic, or any other suitable biocompatible materials. Treatment 45 may include one or more boluses of anti-biotics for treating an infection of patient 4. Treatment 45 may be disposed on or near the surface of IMD 10A, each of which may be behind a weld or door similar to those covering sensors 44. When a bacterial infection is detected by IMD 10A, IMD 10A may open the weld or door covering one or more of the boluses of anti-biotic medication to release anti-biotic medication into the body of patient 4.

FIG. 4B is a conceptual drawing illustrating another example IMD 10B that may include components substantially similar to IMD 10 of FIG. 1. In addition to the components illustrated in FIGS. 1-3, the example of IMD 10B illustrated in FIG. 4B also may include a wafer-scale insulative cover 76, which may help insulate electrical signals passing between electrodes 16A-16D, light detectors 40A, 40B on housing 15B and processing circuitry 50. In some examples, insulative cover 76 may be positioned over an open housing 15 to form the housing for the components of IMD 10B. One or more components of IMD 10B (e.g., antenna 26, light emitter 38, light detectors 40A, 40B, processing circuitry 50, sensing circuitry 52, communication circuitry 54, switching circuitry 58, and/or power source 64) may be formed on a bottom side of insulative cover 76, such as by using flip-chip technology. Insulative cover 76 may be flipped onto a housing 15B. When flipped and placed onto housing 15B, the components of IMD 10B formed on the bottom side of insulative cover 76 may be positioned in a gap 78 defined by housing 15B.

Insulative cover 76 may be configured so as not to interfere with the operation of IMD 10B. For example, insulative cover may include one or more gaps or holes to permit sensors 44 and/or treatment 45 to be exposed to the surrounding tissue of patient 4 when welds or doors 48 are opened. Each of welds or doors 48 may be configured to prevent or limit the exposure of one or more of sensors 44 and/or treatment 45 from the surrounding tissue of patient 4. Additionally, one or more of electrodes 16A-16D may be formed or placed above or on top of insulative cover 76, and electrically connected to switching circuitry 58 through one or more vias (not shown) formed through insulative cover 76. Insulative cover 76 may be formed of sapphire (i.e., corundum), glass, parylene, and/or any other suitable insulating material. Sapphire may be greater than 80% transmissive for wavelengths in the range of about 300 nm to about 4000 nm, and may have a relatively flat profile. In the case of variation, different transmissions at different wavelengths may be compensated for, such as by using a ratiometric approach. In some examples, insulative cover 76 may have a thickness of about 300 micrometers to about 600 micrometers. Housing 15B may be formed from titanium or any other suitable material (e.g., a biocompatible material), and may have a thickness of about 200 micrometers to about 500 micrometers. These materials and dimensions are examples only, and other materials and other thicknesses are possible for devices of this disclosure.

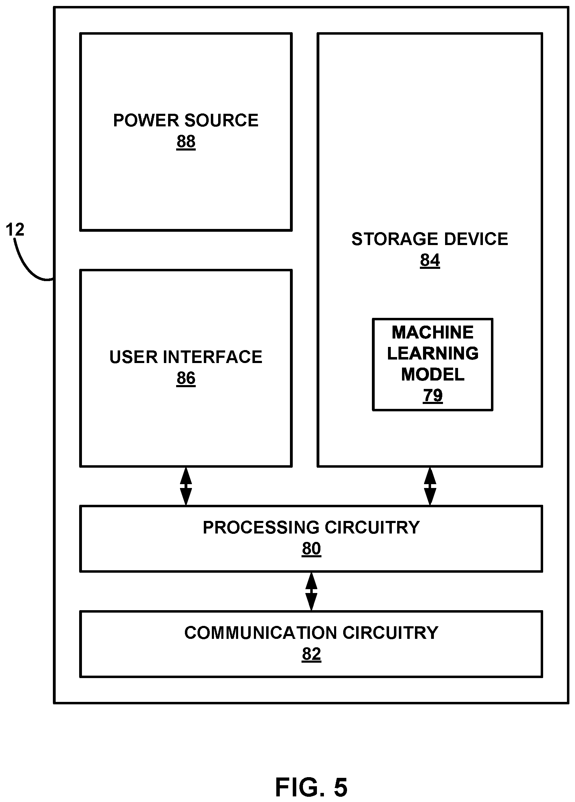

FIG. 5 is a block diagram illustrating an example configuration of components of external device 12, in accordance with one or more techniques of this disclosure. In the example of FIG. 5, external device 12 includes processing circuitry 80, communication circuitry 82, storage device 84, user interface 86, and power source 88.

Processing circuitry 80, in one example, may include one or more processors that are configured to implement functionality and/or process instructions for execution within external device 12. For example, processing circuitry 80 may be capable of processing instructions stored in storage device 84. Processing circuitry 80 may include, for example, microprocessors, DSPs, ASICs, FPGAs, or equivalent discrete or integrated logic circuitry, or a combination of any of the foregoing devices or circuitry. Accordingly, processing circuitry 80 may include any suitable structure, whether in hardware, software, firmware, or any combination thereof, to perform the functions ascribed herein to processing circuitry 80. In some examples, processing circuitry 80 may perform one or more of the techniques of this disclosure.

Communication circuitry 82 may include any suitable hardware, firmware, software or any combination thereof for communicating with another device, such as IMD 10. Under the control of processing circuitry 80, communication circuitry 82 may receive downlink telemetry from, as well as send uplink telemetry to, IMD 10, or another device.

Storage device 84 may be configured to store information within external device 12 during operation. Storage device 84 may include a computer-readable storage medium or computer-readable storage device. In some examples, storage device 84 includes one or more of a short-term memory or a long-term memory. Storage device 84 may include, for example, RAM, dynamic random access memories (DRAM), static random access memories (SRAM), magnetic discs, optical discs, flash memories, or forms of electrically programmable memories (EPROM) or EEPROM. In some examples, storage device 84 is used to store data indicative of instructions for execution by processing circuitry 80. Storage device 84 may be used by software or applications running on external device 12 to temporarily store information during program execution.

Storage device 84 may also store machine learning model 79. In some examples, machine learning model 79 may be used by processing circuitry 80 to determine when to deploy one or more of sensors 44 and/or to instruct patient 4 or a caregiver to deploy one or more biometric sensors as discussed above. In the example where machine learning model 79 is located in storage device 84, external device 12 may generate an instruction for transmission to IMD 10 to deploy one or more biochemical sensors and/or one or more bacterial sensors of sensors 44 and/or output an instruction to patient 4 or a caregiver via user interface 86 to deploy one or more biochemical sensors and/or one or more bacterial sensors.

Data exchanged between external device 12 and IMD 10 may include operational parameters. External device 12 may transmit data including computer readable instructions which, when implemented by IMD 10, may control IMD 10 to change one or more operational parameters (such as to deploy one or more of sensors 44) and/or export collected data. For example, processing circuitry 80 may transmit an instruction to IMD 10 which requests IMD 10 to export collected data (e.g., data corresponding to one or more of optical sensor signals, an accelerometer signal, or other collected data) to external device 12. In turn, external device 12 may receive the collected data from IMD 10 and store the collected data in storage device 84. Additionally, or alternatively, processing circuitry 80 may export instructions to IMD 10 requesting IMD 10 to update one or more operational parameters of IMD 10.

A user, such as a clinician, patient 4, or a caregiver, may interact with external device 12 through user interface 86. User interface 86 includes a display (not shown), such as an LCD or LED display or other type of screen, with which processing circuitry 80 may present information related to IMD 10 (e.g., sensed physiological, biochemical parameters, and/or bacterial parameters). In addition, user interface 86 may include an input mechanism to receive input from the user. The input mechanisms may include, for example, any one or more of buttons, a keypad (e.g., an alphanumeric keypad), a peripheral pointing device, a touch screen, or another input mechanism that allows the user to navigate through user interfaces presented by processing circuitry 80 of external device 12 and provide input. In other examples, user interface 86 also includes audio circuitry for providing audible notifications, instructions or other sounds to patient 4, receiving voice commands from patient 4, or both. Storage device 84 may include instructions for operating user interface 86 and for managing power source 88.

Power source 88 is configured to deliver operating power to the components of external device 12. Power source 88 may include a battery and a power generation circuit to produce the operating power. In some examples, the battery is rechargeable to allow extended operation. Recharging may be accomplished by electrically coupling power source 88 to a cradle or plug that is connected to an alternating current (AC) outlet. In addition, recharging may be accomplished through proximal inductive interaction between an external charger and an inductive charging coil within external device 12. In other examples, traditional batteries (e.g., nickel cadmium or lithium ion batteries) may be used. In addition, external device 12 may be directly coupled to an alternating current outlet to operate.

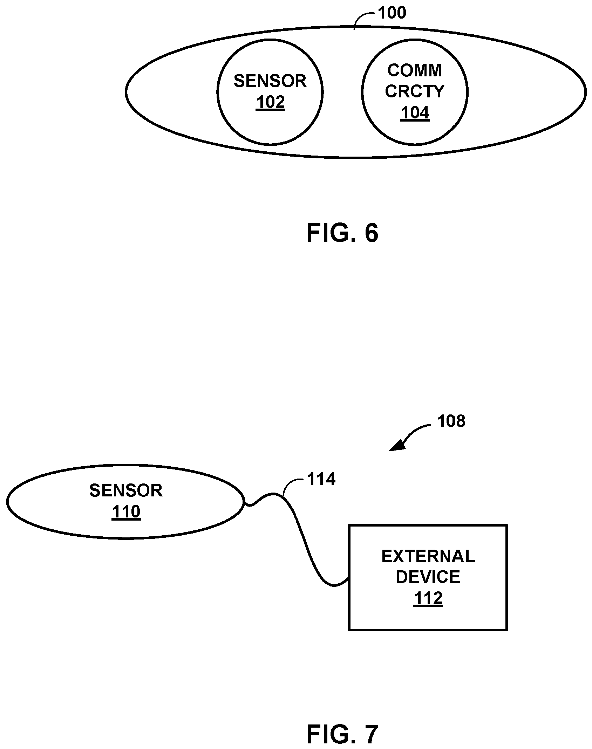

FIG. 6 is a conceptual diagram illustrating an example sensor system which may be deployed by a patient or caregiver according to the techniques of this disclosure. Sensor system 100 may include sensor 102 and communication circuitry 104. In some examples, sensor system 100 may include other components, such as processing circuitry, memory configured to store the sensed signals, additional sensors, or the like. Sensor system 100 may be configured to be injected into patient 4 via a hypodermic needle, a pen device similar to an epi-pen, or any other device configured to deliver sensor system 100 into a body of patient 4. Sensor system 100 may be generally shaped and sized like a grain of rice. For example, sensor system 100 may be 0.5 mm wide and between 3 mm and 5 mm long.

In some examples, sensor 102 may be a biochemical sensor configured to sense one or more biochemical parameters of patient 4. In some examples, sensor 102 may be a bacterial sensor configured to sense one or more bacterial parameters of patient 4. Communication circuitry 104 may be configured to transmit the sensed one or more biochemical parameters or the sensed one or more bacterial parameters of patient 4 to IMD 10 and/or external device 12.

FIG. 7 is a conceptual diagram illustrating another example sensor system which may be deployed by a patient or caregiver according to the techniques of this disclosure. Sensor system 108 may include sensor 110, wire 114, and external device 112. In some examples, external device may be an example of external device 12. Sensor 110 may be a biochemical sensor or bacterial sensor configured to sense one or more biochemical or one or more bacterial parameters of patient 4. Sensor 110 may be communicatively coupled to external device 112 via wire 114 (which may be a pintail) which may remain at least partially external to patient when sensor 110 is inserted into patient 4. Sensor 110 may be similarly sized or smaller than sensor system 100 of FIG. 6. Sensor 110 may be configured to be injected into patient 4 via a hypodermic needle, a pen device similar to an epi-pen, or any other device configured to deliver sensor 110 into a body of patient 4. Sensor signals may travel from sensor 110 over wire 114 to external device 112.

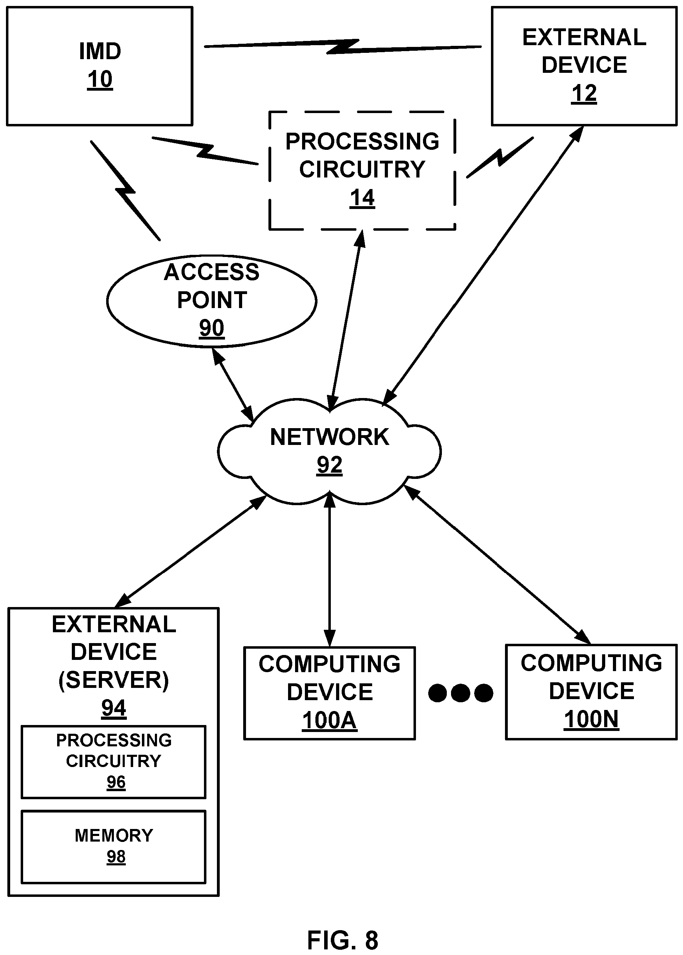

FIG. 8 is a block diagram illustrating an example system that includes an access point 90, a network 92, external computing devices, such as a server 94, and one or more other computing devices 100A-100N, which may be coupled to IMD 10, external device 12, and processing circuitry 14 via network 92, in accordance with one or more techniques described herein. In this example, IMD 10 may use communication circuitry 54 to communicate with external device 12 via a first wireless connection, and to communication with an access point 90 via a second wireless connection. In the example of FIG. 8, access point 90, external device 12, server 94, and computing devices 100A-100N are interconnected and may communicate with each other through network 92.

Access point 90 may include a device that connects to network 92 via any of a variety of connections, such as telephone dial-up, digital subscriber line (DSL), fiber optic, or cable modem connections. In other examples, access point 90 may be coupled to network 92 through different forms of connections, including wired or wireless connections. In some examples, access point 90 may be a user device, such as a tablet or smartphone, that may be co-located with the patient. As discussed above, IMD 10 may be configured to transmit data, such an indication that a heart failure decompensation event is impending, biochemical sensor or bacterial sensor signals, optical sensor signals, an accelerometer signal, or other data collected by IMD 10 to external device 12. In addition, access point 90 may interrogate IMD 10, such as periodically or in response to a command from the patient or network 92, in order to retrieve parameter values determined by processing circuitry 50 of IMD 10, or other operational or patient data from IMD 10. Access point 90 may then communicate the retrieved data to server 94 via network 92.

In some cases, server 94 may be configured to provide a secure storage site for data that has been collected from IMD 10, and/or external device 12, such as physiological parameters and/or biochemical parameters of patient 4. In some cases, server 94 may assemble data in web pages or other documents for viewing by trained professionals, such as clinicians, via computing devices 100A-100N. One or more aspects of the illustrated system of FIG. 8 may be implemented with general network technology and functionality, which may be similar to that provided by the Medtronic CareLink^{®} Network developed by Medtronic, Inc.

Server 94 may include processing circuitry 96. Processing circuitry 96 may include fixed function circuitry and/or programmable processing circuitry. Processing circuitry 96 may include any one or more of a microprocessor, a controller, a DSP, an ASIC, an FPGA, or equivalent discrete or analog logic circuitry. In some examples, processing circuitry 96 may include multiple components, such as any combination of one or more microprocessors, one or more controllers, one or more DSPs, one or more ASICs, or one or more FPGAs, as well as other discrete or integrated logic circuitry. The functions attributed to processing circuitry 96 herein may be embodied as software, firmware, hardware or any combination thereof. In some examples, processing circuitry 96 may perform one or more techniques described herein, e.g., application of a machine learning model to patient parameter data to determine when IMD 10 should activate sensor(s) 44 and/or to determine a risk level of a heart failure event.

Server 94 may include memory 98. Memory 98 includes computer-readable instructions that, when executed by processing circuitry 96, cause IMD 10 and processing circuitry 96 to perform various functions attributed to IMD 10 and processing circuitry 96 herein. Memory 98 may include any volatile, non-volatile, magnetic, optical, or electrical media, such as RAM, ROM, NVRAM, EEPROM, flash memory, or any other digital media. Memory 98 may store a machine learning model.

In some examples, one or more of computing devices 100A-100N (e.g., device 100A) may be a tablet or other smart device located with a clinician, by which the clinician may program, receive alerts from, and/or interrogate IMD 10. For example, the clinician may access data corresponding to physiological parameters, biochemical parameters, and/or bacterial parameters of patient 4 determined by IMD 10, external device 12, processing circuitry 14, or server 94 through device 100A, such as when patient 4 is in between clinician visits, to check on a status of a medical condition, such as heart failure. In some examples, the clinician may enter instructions for a medical intervention for patient 4 into an app in device 100A, such as based on a status of a patient condition determined by IMD 10, external device 12, processing circuitry 14, or any combination thereof, or based on other patient data known to the clinician. Device 100A then may transmit the instructions for medical intervention to another of computing devices 100A-100N (e.g., device 100B) located with patient 4 or a caregiver of patient 4. For example, such instructions for medical intervention may include an instruction to change a drug dosage, timing, or selection, to schedule a visit with the clinician, or to seek medical attention. In further examples, device 100B may generate an indication for output, such as an alert to patient 4 based on a status of a medical condition of patient 4 determined by IMD 10, which may enable patient 4 proactively to seek medical attention prior to receiving instructions for a medical intervention. In this manner, patient 4 may be empowered to take action, as needed, to address his or her medical status, which may help improve clinical outcomes for patient 4.

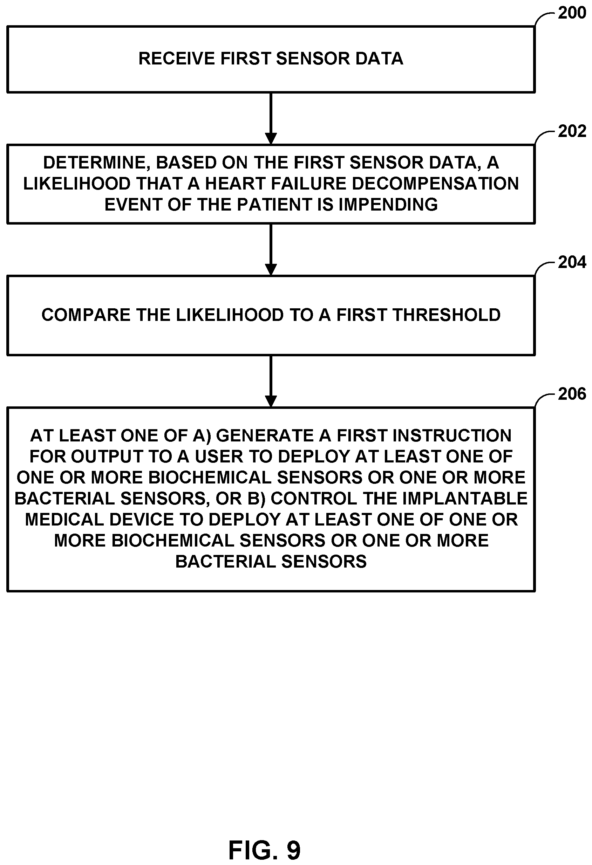

FIG. 9 is a flow diagram illustrating an example of biochemical sensor or bacterial sensor deployment techniques according to one or more aspects of this disclosure. The example of FIG. 9 is discussed with respect to IMD 10 of FIG. 3, but these techniques may be implemented in any combination of devices of system 2 (FIG. 1) or of FIG. 8.

Processing circuitry 50 may receive first sensor data (200). For example, medical device system 2 may include one or more sensors (e.g., sensing circuitry 52 and/or sensors 62) configured to generate the first sensor data. Processing circuitry 50 may receive sensor data from sensing circuitry 52 and/or sensors 62. The first sensor data may include physiological parameters such as impedance (e.g., OptiVol^{™} fluid index), patient activity, tachycardia/fibrillation burden, ventricular rate during tachycardia/fibrillation, percentage of ventricular pacing, shocks, treated ventricular tachycardia/ventricular fibrillation, night ventricular rate, heart rate variability, and/or other physiological parameters.

Processing circuitry 50 may determine, based on the first sensor data, a likelihood that a heart failure decompensation event of patient 4 is impending (202). For example, processing circuitry 50 may execute an algorithm that is configured to calculate a likelihood that a heart failure decompensation event of patient 4 is impending based on the first sensor data.

Processing circuitry 50 may compare the likelihood to a first threshold (204). For example, processing circuitry may retrieve the first threshold from thresholds 47 and compare the likelihood to the first threshold. Based on the likelihood satisfying the first threshold, processing circuitry 50 may at least one of a) generating a first instruction for output to a user to deploy at least one of one or more biochemical sensors or one or more bacterial sensors, or b) control IMD 10 to deploy at least one of one or more biochemical or one or more bacterial sensors (206). For example, processing circuitry 50 may generate a first instruction for output to patient 4 or a caregiver of patient 4 to deploy one or more biochemical sensors, one or more bacterial sensors, or both one or more biochemical sensors and one or more bacterial sensors. Such an instruction may be output to external device 12 for review by a user, such as patient 4 or a caregiver of patient 4. In some examples, the instruction may be displayed on external device 12. Alternatively, or additionally, processing circuitry 50 may control IMD 10 to deploy one or more biochemical sensors, one or more bacterial sensors, or both one or more biochemical sensors and one or more bacterial sensors.

In some examples, processing circuitry 50 may receive at least one of biochemical sensor data from the one or more biochemical sensors or bacterial sensor data from the one or more bacterial sensors and update the likelihood that a heart failure decompensation event is impending based on at least one of the biochemical sensor data or the bacterial sensor data. In some examples, processing circuitry 50 may compare the updated likelihood to a second threshold and generate, based on the updated likelihood that the heart failure decompensation event is impending meeting the second threshold, a first indication for output to a user (e.g., a clinician, patient 4, or a caregiver of patient 4). In some examples, the first indication includes at least one of an indication to seek medical attention, an indication of the updated likelihood that the heart failure decompensation event is impending, or a proposed treatment of the patient. In some examples, processing circuitry 50 may output the first indication to the user. For example, processing circuitry 50 may control communication circuitry 54 to transmit the first indication to external device 12 for review by patient 4, a caregiver of patient 4, or a clinician. External device 12 may, for example, display the first indication on user interface 86 (FIG. 5).

In some examples, IMD 10 includes an insertable cardiac monitor. In some examples, the one or more biochemical sensors or the one or more bacterial sensors are disposed on IMD 10. In some examples, IMD 10 includes one or more boluses of anti-biotic medication disposed on IMD 10 and processing circuitry 50 determines that the bacterial sensor data indicates a bacterial infection and controls IMD 10 to release at least one of the one or more boluses of anti-biotic medication based on the determination that the bacterial sensor data indicates the bacterial infection.

In some examples, at least one of the one or more biochemical sensors or at least one of the one or more bacterial sensors are insertable into the patient by the patient or a caregiver. In some examples, processing circuitry 50 may at least one of generate a second instruction for output to a user to deploy one or more biochemical or bacterial sensors according to a schedule, or control IMD 10 to deploy one or more biochemical or bacterial sensors according to the schedule. In some examples, the schedule is at least one of: predetermined; programmed by a clinician; based on a change in pattern of at least one of sensed biochemical parameters, bacterial parameters, or physiological parameters over time; or determined by the processing circuitry executing a machine learning model.

In some examples, processing circuitry 50 may receive at least one of biochemical sensor data from the one or more biochemical sensors or bacterial sensor data from the one or more bacterial sensors and generate a second indication for output to a clinician, the second indication comprising at least one of the biochemical sensor data or the bacterial sensor data.

In some examples, processing circuitry 50 may generate a score based on at least one of the first sensor data, the biochemical sensor data, or the bacterial sensor data and output the score to a clinician. For example, processing circuitry 50 may control communication circuitry 54 to output the score to external device 12. External device 12 may, for example, display the score on user interface 86 (FIG. 5). In some examples, processing circuitry 50 may compare the score to a previous score and determine that the score is different than the previous score. In some examples, processing circuitry 50 may output a third indication to the clinician, the third indication including at least one or information relating to the change in the score from the previous score, a source of the change in the score from the previous score, or suggested treatment options.

In some examples, the first sensor data comprises a plurality of sensed physiological parameters and to determine the likelihood that a heart failure decompensation event of a patient is impending, processing circuitry 50 compares each of the plurality of sensed physiological parameters to parameter specific thresholds and applies a Bayesian probabilistic model to results of the comparisons. In some examples, the plurality of sensed physiological parameters comprise at least two of a fluid index, patient activity, atrial tachycardia (AT)/atrial fibrillation (AF) burden, ventricular rate during AT/AF, percentage of ventricular pacing, shocks, treated ventricular tachycardia/ventricular fibrillation, night ventricular rate, heart rate variability, heart sounds, or lung sounds.,

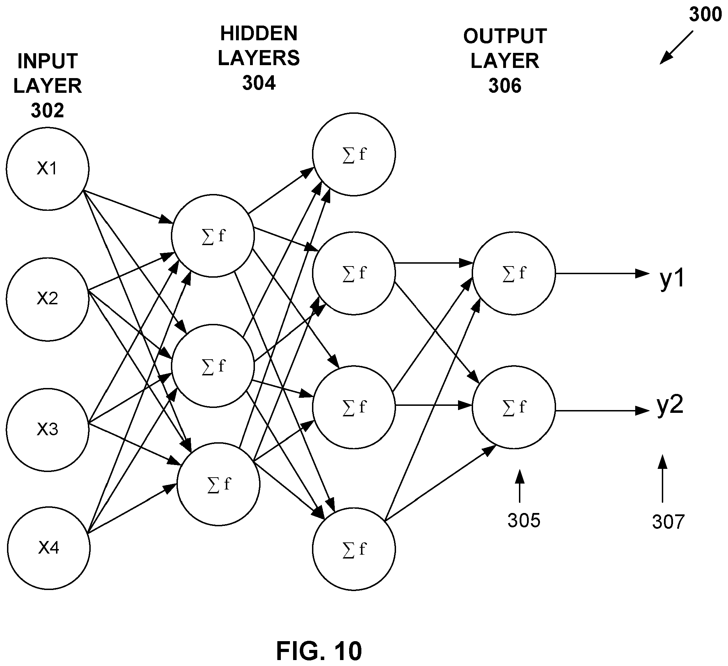

FIG. 10 is a conceptual diagram illustrating an example machine learning model 300 configured to determine one or more output values based on patient parameter data as described herein. Machine learning model 300 may correspond to machine learning models 46 and 79. Machine learning model 300 is an example of a deep learning model, or deep learning algorithm, trained to determine whether a particular set of patient parameter data indicates a condition of the patient meriting a system communication as described herein. One or more of IMD 10, external device 12, or server 94 may train, store, and/or utilize machine learning model 300, but other devices may apply inputs associated with a particular patient to machine learning model 300 in other examples. Some non-limiting examples of machine learning techniques include Bayesian probability models, Support Vector Machines, K-Nearest Neighbor algorithms, and Multi-layer Perceptron.

As shown in the example of FIG. 10, machine learning model 300 may include three layers. These three layers include input layer 302, hidden layer 304, and output layer 306. Output layer 306 comprises the output from the transfer function 305 of output layer 306. Input layer 302 represents each of the input values X1 through X4 provided to machine learning model 300. The number of inputs may be less than or greater than 4, including much greater than 4, e.g., hundreds or thousands. In some examples, the input values may any of the of values input into a machine learning model, as described above. In some examples, input values may include parameter values described herein, e.g., impedance or fluid index, night heart rate, heart rate variability, and arrhythmia metrics. In addition, in some examples input values of machine learning model 300 may include additional data, such as analyte or bacteria values.

Each of the input values for each node in the input layer 302 is provided to each node of hidden layer 304. In the example of FIG. 10, hidden layers 304 include two layers, one layer having four nodes and the other layer having three nodes, but fewer or greater number of nodes may be used in other examples. Each input from input layer 302 is multiplied by a weight and then summed at each node of hidden layers 304. During training of machine learning model 300, the weights for each input are adjusted to establish the relationship between the inputs determining whether a particular set of inputs represents a health event and/or determining a score indicative of whether a set of inputs may be representative of a health event, e.g., a risk level of heart failure decompensation. In some examples, one hidden layer may be incorporated into machine learning model 300, or three or more hidden layers may be incorporated into machine learning model 300, where each layer includes the same or different number of nodes.

The result of each node within hidden layers 304 is applied to the transfer function of output layer 306. The transfer function may be liner or non-linear, depending on the number of layers within machine learning model 300. Example non-linear transfer functions may be a sigmoid function or a rectifier function. The output 307 of the transfer function may be or a score indicative of a risk level (e.g., likelihood or probability) of heart failure decompensation. By applying the patient parameter data to a machine learning model, such as machine learning model 300, processing circuitry of system 2 is able to determine a patient is experiencing or will soon experience a health event, such as heart failure decompensation, with great accuracy, specificity, and sensitivity. This may facilitate activation of sensors, alerts, or other actions as described herein.

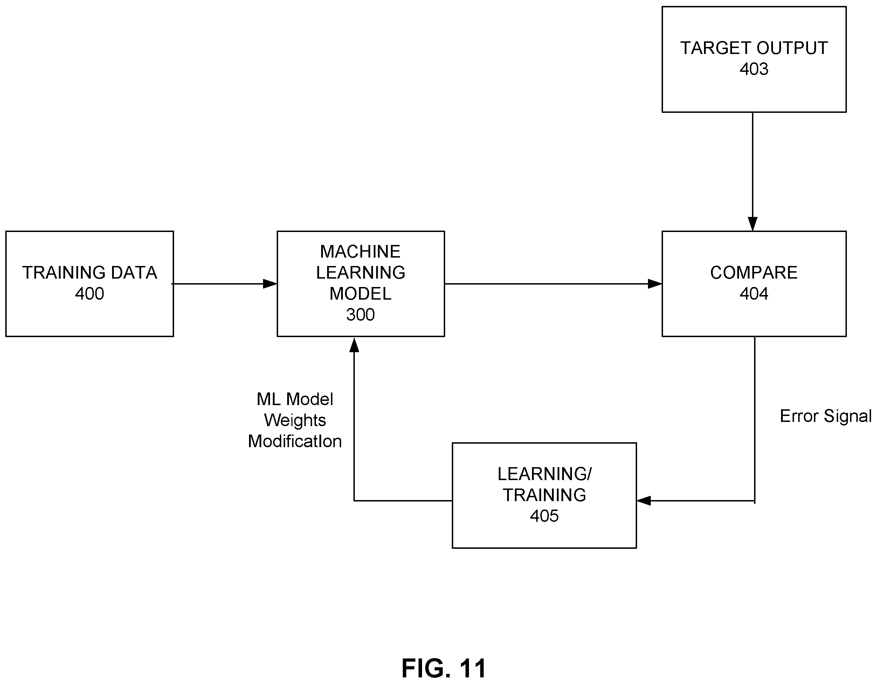

FIG. 11 is an example of a machine learning model 300 being trained using supervised and/or reinforcement learning techniques. Machine learning model 300 may be implemented using any number of models for supervised and/or reinforcement learning, such as but not limited to, an artificial neural network, a decision tree, naive Bayes network, support vector machine, or k-nearest neighbor model, to name only a few of the examples discussed above. In some examples, processing circuitry one or more of IMD 10, external device 12, and/or server 94 initially trains the machine learning model 300 based on training set data 400 including numerous instances of input data corresponding to health events, such as heart failure decompensation, and non-health events, e.g., as labeled by an expert. A prediction or classification by the machine learning model 300 may be compared 404 to the target output 403, e.g., as determined based on the label. Based on an error signal representing the comparison, the processing circuitry implementing a learning/training function 405 may send or apply a modification to weights of machine learning model 300 or otherwise modify/update the machine learning model 300. For example, one or more of IMD 10, external device 12, and/or server 94 may, for each training instance in the training set 400, modify machine learning model 300 to change a score generated by the machine learning model 300 in response to data applied to the machine learning model 300.

The techniques described in this disclosure may be implemented, at least in part, in hardware, software, firmware, or any combination thereof. For example, various aspects of the techniques may be implemented within one or more microprocessors, DSPs, ASICs, FPGAs, or any other equivalent integrated or discrete logic QRS circuitry, as well as any combinations of such components, embodied in external devices, such as clinician or patient programmers, stimulators, or other devices. The terms "processor" and "processing circuitry" may generally refer to any of the foregoing logic circuitry, alone or in combination with other logic circuitry, or any other equivalent circuitry, and alone or in combination with other digital or analog circuitry.

For aspects implemented in software, at least some of the functionality ascribed to the systems and devices described in this disclosure may be embodied as instructions on a computer-readable storage medium such as RAM, FRAM, DRAM, SRAM, magnetic discs, optical discs, flash memories, or forms of EPROM or EEPROM. The instructions may be executed to support one or more aspects of the functionality described in this disclosure.

In addition, in some aspects, the functionality described herein may be provided within dedicated hardware and/or software modules. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components. Also, the techniques could be fully implemented in one or more circuits or logic elements. The techniques of this disclosure may be implemented in a wide variety of devices or apparatuses, including an IMD, an external programmer, a combination of an IMD and external programmer, an integrated circuit (IC) or a set of ICs, and/or discrete electrical circuitry, residing in an IMD and/or external programmer.

## Claims

1. A medical device system (2) comprising:
an implantable medical device (10) configured to sense one or more physiological signals and determine first sensor data based on the one or more physiological signals; and
processing circuitry (14) communicatively coupled to the memory, the processing circuitry being configured to:
receive the first sensor data;
determine, based on the first sensor data, a likelihood that a heart failure decompensation event of a patient is impending;
compare the likelihood to a first threshold; and
based on the likelihood satisfying the first threshold, at least one of a) generate a first instruction for output to a user to deploy at least one of one or more biochemical sensors or one or more bacterial sensors (44), or b) control the implantable medical device or another implantable medical device to automatically deploy at least one of one or more biochemical sensors or one or more bacterial sensors (44).

2. The medical device system of claim 1, wherein the processing circuitry is further configured to output the first instruction to the user.

3. The medical device system of claim 1 or 2, wherein the processing circuitry is further configured to:
receive at least one of biochemical sensor data from the one or more biochemical sensors or bacterial sensor data from the one or more bacterial sensors; and
update the likelihood that a heart failure decompensation event is impending based on at least one of the biochemical sensor data or the bacterial sensor data.

4. The medical device system of claim 3, wherein the processing circuitry is further configured to:
compare the updated likelihood to a second threshold; and
based on the updated likelihood meeting the second threshold, generate a first indication for output to a user.

5. The medical device system of claim 4, wherein the first indication comprises at least one of:
an indication to seek medical attention;
an indication of the updated likelihood that the heart failure decompensation event is impending; or
a proposed treatment of the patient.

6. The medical device system of claim 4 or 5, wherein the processing circuitry is further configured to output the first indication to the user.

7. The medical device system of any of claims 1-6, wherein the implantable medical device comprises an insertable cardiac monitor.

8. The medical device system of claim 7, wherein the insertable cardiac monitor comprises:
a housing (15) configured for subcutaneous implantation in a patient, the housing having a length between 40 millimeters (mm) and 60 mm between a first end and a second end, a width less than the length, and a depth less than the width;
a first electrode (16A) at or proximate to the first end; and
a second electrode (16B) at or proximate to the second end,
wherein the insertable cardiac monitor is configured to sense at least one of the one or more physiological signals via the first electrode and the second electrode.

9. The medical device system of any one or more of claims 1-8, further comprising one or more boluses of anti-biotic medication disposed on the implantable medical device, wherein the one or more bacterial sensors are configured to generate bacterial sensor data, and wherein the processing circuitry is further configured to:
determine that the bacterial sensor data indicates a bacterial infection; and
control the implantable medical device to release at least one of the one or more boluses of anti-biotic medication based on the determination that the bacterial sensor data indicates the bacterial infection.

10. The medical device system of any of claims 1-9, wherein at least one of the one or more biochemical sensors or at least one of the one or more bacterial sensors are insertable into the patient by the patient or a caregiver.

11. The medical device system of any of claims 1-10, wherein the processing circuitry is further configured to at least one of:
generate a second instruction for output to a user to deploy one or more biochemical or bacterial sensors according to a schedule, or
control the implantable medical device to deploy one or more biochemical or bacterial sensors according to the schedule.

12. The medical device system of claim 11, wherein the schedule is at least one of:
predetermined;
programmed by a clinician;
based on a change in pattern of at least one of sensed biochemical parameters, bacterial parameters, or physiological parameters over time; or
determined by the processing circuitry executing a machine learning model.

13. The medical device system of claim 11 or claim 12, wherein the processing circuitry is further configured to:
receive at least one of biochemical sensor data from the one or more biochemical sensors or bacterial sensor data from the one or more bacterial sensors; and
generate a second indication for output to a clinician, the second indication comprising at least one of the biochemical sensor data or the bacterial sensor data.

14. The medical device system of any of claims 1-13, wherein the processing circuitry is further configured to:
generate a score based on at least one of the first sensor data, the biochemical sensor data, or the bacterial sensor data; and
output the score to a clinician, optionally,
wherein the processing circuitry is further configured to:
compare the score to a previous score;
determine that the score is different than the previous score; and
output a third indication for review by the clinician, the third indication comprising at least one of information relating to the change in the score from the previous score, a source of the change in the score from the previous score, or suggested treatment options.

15. The medical device of any of claims 1-14, wherein the first sensor data comprises a plurality of sensed physiological parameters and wherein as part of determining the likelihood that a heart failure decompensation event of a patient is impending, the processing circuitry is configured to:
compare each of the plurality of sensed physiological parameters to parameter specific thresholds; and
apply a Bayesian probabilistic model to results of the comparisons, optionally, wherein the plurality of sensed physiological parameters comprise at least two of a fluid index, patient activity, atrial tachycardia (AT)/atrial fibrillation (AF) burden, ventricular rate during AT/AF, percentage of ventricular pacing, shocks, treated ventricular tachycardia/ventricular fibrillation, night ventricular rate, heart rate variability, heart sounds, or lung sounds.

## Patentansprüche

1. Medizinisches Vorrichtungssystem (2) umfassend:
eine implantierbare medizinische Vorrichtung (10), die dazu ausgelegt ist, ein oder mehrere physiologische Signale zu erfassen und erste Sensordaten basierend auf den ein oder mehreren physiologischen Signalen zu bestimmen; und
eine Verarbeitungsschaltung (14), die kommunikativ mit dem Speicher gekoppelt ist, wobei die Verarbeitungsschaltung hierzu ausgelegt ist:
Empfangen der ersten Sensordaten;
Bestimmen, basierend auf den ersten Sensordaten, einer Wahrscheinlichkeit, dass ein Herzinsuffizienz-Dekompensationsereignis eines Patienten bevorsteht;
Vergleichen der Wahrscheinlichkeit mit einem ersten Schwellwert; und
basierend darauf, dass die Wahrscheinlichkeit den ersten Schwellwert erfüllt, wenigstens eines von a) Generieren einer ersten Anweisung zur Ausgabe an einen Benutzer, um wenigstens eines von einem oder mehreren biochemischen Sensoren oder einem oder mehreren bakteriellen Sensoren (44) zu nutzen oder b) Steuern der implantierbaren medizinischen Vorrichtung oder einer anderen implantierbaren medizinischen Vorrichtung, um wenigstens eines von einem oder mehreren biochemischen Sensoren oder einem oder mehreren bakteriellen Sensoren (44) automatisch zu nutzen.

2. Medizinisches Vorrichtungssystem nach Anspruch 1, wobei die Verarbeitungsschaltung ferner dazu ausgelegt ist, die erste Anweisung an den Benutzer auszugeben.

3. Medizinisches Vorrichtungssystem nach Anspruch 1 oder 2, wobei die Verarbeitungsschaltung ferner hierzu ausgelegt ist:
Empfangen von wenigstens einem von biochemischen Sensordaten von den ein oder mehreren biochemischen Sensoren oder bakteriellen Sensordaten von den ein oder mehreren bakteriellen Sensoren; und
Aktualisieren der Wahrscheinlichkeit, dass ein Herzinsuffizienz-Dekompensationsereignis bevorsteht, basierend auf wenigstens einem von den biochemischen Sensordaten oder den bakteriellen Sensordaten.

4. Medizinisches Vorrichtungssystem nach Anspruch 3, wobei die Verarbeitungsschaltung ferner hierzu ausgelegt ist:
Vergleichen der aktualisierten Wahrscheinlichkeit mit einem zweiten Schwellwert; und
basierend darauf, dass die aktualisierte Wahrscheinlichkeit den zweiten Schwellwert erfüllt, Generieren einer ersten Angabe zur Ausgabe an einen Benutzer.

5. Medizinisches Vorrichtungssystem nach Anspruch 4, wobei die erste Angabe wenigstens eines hiervon umfasst:
eine Angabe, ärztliche Hilfe in Anspruch zu nehmen;
eine Angabe der aktualisierten Wahrscheinlichkeit, dass das Herzinsuffizienz-Dekompensationsereignis bevorsteht; oder
eine vorgeschlagene Behandlung des Patienten.

6. Medizinisches Vorrichtungssystem nach Anspruch 4 oder 5, wobei die Verarbeitungsschaltung ferner dazu ausgelegt ist, die erste Angabe an den Benutzer auszugeben.

7. Medizinisches Vorrichtungssystem nach einem der Ansprüche 1-6, wobei die implantierbare medizinische Vorrichtung einen einsetzbaren Herzmonitor umfasst.

8. Medizinisches Vorrichtungssystem nach Anspruch 7, wobei der einsetzbare Herzmonitor dies umfasst:
ein Gehäuse (15), das zur subkutanen Implantation in einen Patienten ausgelegt ist, wobei das Gehäuse eine Länge zwischen 40 Millimeter (mm) und 60 mm zwischen einem ersten Ende und einem zweiten Ende aufweist, sowie eine Breite, die kleiner als die Länge ist, und eine Tiefe, die kleiner als die Breite ist;
eine erste Elektrode (16A) an oder nahe dem ersten Ende; und
eine zweite Elektrode (16B) an oder nahe dem zweiten Ende,
wobei der einsetzbare Herzmonitor dazu ausgelegt ist, wenigstens eines der ein oder mehreren physiologischen Signale über die erste Elektrode und die zweite Elektrode zu erfassen.

9. Medizinisches Vorrichtungssystem nach einem oder mehreren der Ansprüche 1-8, ferner umfassend einen oder mehrere Boli einer antibiotischen Medikation, die sich in der implantierbaren medizinischen Vorrichtung befindet, wobei die ein oder mehreren bakteriellen Sensoren dazu ausgelegt sind, bakterielle Sensordaten zu generieren, und wobei die Verarbeitungsschaltung ferner hierzu ausgelegt ist:
Bestimmen, dass die bakteriellen Sensordaten eine bakterielle Infektion angeben; und
Steuern der implantierbaren medizinischen Vorrichtung, um, basierend auf der Bestimmung, dass die bakteriellen Sensordaten die bakterielle Infektion angeben, wenigstens einen der ein oder mehreren Boli einer antibiotischen Medikation freizusetzen.

10. Medizinisches Vorrichtungssystem nach einem der Ansprüche 1-9, wobei wenigstens einer der ein oder mehreren biochemischen Sensoren oder wenigstens einer der ein oder mehreren bakteriellen Sensoren von dem Patienten oder einem Betreuer in den Patienten eingesetzt werden kann.

11. Medizinisches Vorrichtungssystem nach einem der Ansprüche 1-10, wobei die Verarbeitungsschaltung ferner zu wenigstens einem hiervon ausgelegt ist:
Generieren einer zweiten Anweisung zur Ausgabe an einen Benutzer, um einen oder mehrere biochemische oder bakterielle Sensoren gemäß einem Zeitplan zu nutzen, oder Steuern der implantierbaren medizinischen Vorrichtung, um einen oder mehrere biochemische oder bakterielle Sensoren gemäß dem Zeitplan zu nutzen.

12. Medizinisches Vorrichtungssystem nach Anspruch 11, wobei der Zeitplan wenigstens eines hiervon ist:
vorbestimmt;
von einem Kliniker programmiert;
auf einer Änderung im Muster wenigstens eines erfassten biochemischen Parameters, bakteriellen Parameters oder physiologischen Parameters über die Zeit basierend; oder durch die Verarbeitungsschaltung bestimmt, die ein Maschinenlernmodell ausführt.

13. Medizinisches Vorrichtungssystem nach Anspruch 11 oder Anspruch 12, wobei die Verarbeitungsschaltung ferner hierzu ausgelegt ist:
Empfangen von wenigstens einem von biochemischen Sensordaten von den ein oder mehreren biochemischen Sensoren oder bakteriellen Sensordaten von den ein oder mehreren bakteriellen Sensoren; und
Generieren einer zweiten Angabe zur Ausgabe an einen Kliniker, wobei die zweite Angabe wenigstens eines von den biochemischen Sensordaten oder den bakteriellen Sensordaten umfasst.

14. Medizinische Vorrichtung nach einem der Ansprüche 1-13, wobei die Verarbeitungsschaltung ferner hierzu ausgelegt:
Generieren einer Bewertung, die auf wenigstens einem von den ersten Sensordaten, den biochemischen Sensordaten oder den bakteriellen Sensordaten basiert; und
Ausgeben der Bewertung an einen Kliniker, optional,
wobei die Verarbeitungsschaltung ferner hierzu ausgelegt ist:
Vergleichen der Bewertung mit einer vorherigen Bewertung; Bestimmen, dass die Bewertung von der vorherigen Bewertung verschieden ist; und
Ausgeben einer dritten Angabe zur Überprüfung durch den Kliniker, wobei die dritte Angabe wenigstens eines von Informationen zur Änderung der Bewertung gegenüber der vorherigen Bewertung, eine Quelle für die Änderung der Bewertung gegenüber der vorherigen Bewertung oder vorgeschlagene Behandlungsoptionen umfasst.

15. Medizinische Vorrichtung nach einem der Ansprüche 1-14, wobei die ersten Sensordaten mehrere erfasste physiologische Parameter umfassen und wobei im Rahmen des Bestimmens der Wahrscheinlichkeit, dass ein Herzinsuffizienz-Dekompensationsereignis eines Patienten bevorsteht, die Verarbeitungsschaltung hierzu ausgelegt ist:
Vergleichen jedes der mehreren erfassten physiologischen Parameter mit parameterspezifischen Schwellwerten; und
Anwenden eines Bayesschen probabilistischen Modells auf Ergebnisse der Vergleiche, wobei, optional, die mehreren erfassten physiologischen Parameter wenigstens zwei hiervon umfassen: Fluidindex, Patientenaktivität, atriale Tachykardiebelastung (AT-Belastung)/atriale Fibrillationsbelastung (AF-Belastung), ventrikuläre Rate während einer AT/AF, Prozentsatz einer ventrikulären Stimulation, Schocks, behandelte ventrikuläre Tachykardie/ventrikuläre Fibrillation, nächtliche ventrikuläre Rate, Herzfrequenzvariabilität, Herzgeräusche oder Lungengeräusche.

## Revendications

1. Système de dispositif médical (2) comprenant :
un dispositif médical implantable (10) configuré pour détecter un ou plusieurs signaux physiologiques et déterminer des premières données de capteur sur la base du ou des signaux physiologiques ; et
un circuit de traitement (14) couplé en communication à la mémoire, le circuit de traitement étant configuré pour :
recevoir les données du premier capteur ;
déterminer, sur la base des données du premier capteur, une probabilité qu'un événement de décompensation d'insuffisance cardiaque d'un patient soit imminent ;
comparer la probabilité par rapport à un premier seuil ; et
sur la base de la probabilité satisfaisant au premier seuil, a) générer une première instruction destinée à délivrer à un utilisateur pour déployer au moins un ou plusieurs capteurs biochimiques et/ou un ou plusieurs capteurs bactériens (44), et/ou b) commander le dispositif médical implantable ou un autre dispositif médical implantable pour déployer automatiquement un ou plusieurs capteurs biochimiques et/ou un ou plusieurs capteurs bactériens (44).

2. Système de dispositif médical selon la revendication 1, dans lequel le circuit de traitement est en outre configuré pour délivrer la première instruction à l'utilisateur.

3. Système de dispositif médical selon la revendication 1 ou 2, dans lequel le circuit de traitement est en outre configuré pour :
recevoir des données de capteur biochimique provenant du ou des capteurs biochimiques et/ou des données de capteur bactérien provenant du ou des capteurs bactériens ; et
mettre à jour la probabilité qu'un événement de décompensation d'insuffisance cardiaque soit imminent sur la base des données du capteur biochimique et/ou des données du capteur bactérien.

4. Système de dispositif médical selon la revendication 3, dans lequel le circuit de traitement est en outre configuré pour :
comparer la probabilité mise à jour à un second seuil ; et
sur la base de la probabilité mise à jour satisfaisant au second seuil, générer une première indication à délivrer à un utilisateur.

5. Système de dispositif médical selon la revendication 4, dans lequel la première indication comprend au moins l'une parmi :
une indication pour demander des soins médicaux ;
une indication de la probabilité mise à jour que l'événement de décompensation d'insuffisance cardiaque soit imminent ; ou
une proposition de traitement du patient.

6. Système de dispositif médical selon la revendication 4 ou 5, dans lequel le circuit de traitement est en outre configuré pour délivrer la première indication à l'utilisateur.

7. Système de dispositif médical selon l'une quelconque des revendications 1 à 6, dans lequel le dispositif médical implantable comprend un moniteur cardiaque insérable.

8. Système de dispositif médical selon la revendication 7, dans lequel le moniteur cardiaque insérable comprend :
un boîtier (15) configuré pour une implantation sous-cutanée chez un patient, le boîtier ayant une longueur comprise entre 40 millimètre (mm) et 60 mm entre une première extrémité et une seconde extrémité, une largeur inférieure à la longueur, et une profondeur inférieure à la largeur ;
une première électrode (16A) au niveau de la première extrémité ou à proximité de celle-ci ; et
une seconde électrode (16B) au niveau de la seconde extrémité ou à proximité de celle-ci,
dans lequel le moniteur cardiaque insérable est configuré pour détecter l'un du ou des signaux physiologiques par l'intermédiaire de la première électrode et de la seconde électrode.

9. Système de dispositif médical selon l'une quelconque des revendications 1 à 8, comprenant en outre un ou plusieurs bolus de médicament antibiotique disposés sur le dispositif médical implantable, dans lequel le ou les capteurs bactériens sont configurés pour générer des données de capteur bactérien, et dans lequel le circuit de traitement est en outre configuré pour :
déterminer que les données du capteur bactérien indiquent une infection bactérienne ; et
commander le dispositif médical implantable pour libérer au moins l'un du ou des bolus de médicament antibiotique en fonction de la détermination que les données du capteur bactérien indiquent l'infection bactérienne.

10. Système de dispositif médical selon l'une quelconque des revendications 1 à 9, dans lequel au moins l'un des un ou plusieurs capteurs biochimiques ou au moins l'un des un ou plusieurs capteurs bactériens peut être inséré dans le patient par le patient ou un soignant.

11. Système de dispositif médical selon l'une quelconque des revendications 1 à 10, dans lequel le circuit de traitement est en outre configuré pour :
générer une seconde instruction destinée à délivrer à un utilisateur pour déployer un ou plusieurs capteurs biochimiques ou bactériens selon un calendrier, et/ou commander le dispositif médical implantable pour déployer un ou plusieurs capteurs biochimiques ou bactériens selon le calendrier.

12. Dispositif médical selon la revendication 11, dans lequel le calendrier est :
prédéterminé ;
et/ou programmé par un clinicien ;
et/ou basé sur un changement de modèle d'au moins un de paramètres biochimiques, paramètres bactériens ou paramètres physiologiques détectés dans le temps ; et/ou déterminé par le circuit de traitement exécutant un modèle d'apprentissage automatique.

13. Système de dispositif médical selon la revendication 11 ou la revendication 12, dans lequel le circuit de traitement est en outre configuré pour :
recevoir des données de capteur biochimique provenant du ou des capteurs biochimiques et/ou des données de capteur bactérien provenant du ou des capteurs bactériens ; et
générer une seconde indication à délivrer à un clinicien, la seconde indication comprenant les données de capteurs biochimiques ou les données de capteurs bactériens.

14. Système de dispositif médical selon l'une quelconque des revendications 1 à 13, dans lequel le circuit de traitement est en outre configuré pour :
générer un score basé sur les premières données de capteurs, les données du capteur biochimique et/ou les données du capteur bactérien ; et
délivrer le score à un clinicien, éventuellement,
le circuit de traitement étant en outre configuré pour :
comparer le score à un score précédent ;
déterminer que le score est différent du score précédent ; et
délivrer une troisième indication pour examen par le clinicien, la troisième indication comprenant des informations relatives au changement du score par rapport au score précédent, et/ou une source du changement du score par rapport au score précédent, et ou des options de traitement suggérées.

15. Dispositif médical selon l'une quelconque des revendications 1 à 14, dans lequel les premières données de capteur comprennent une pluralité de paramètres physiologiques détectés et dans lequel, dans le cadre de la détermination de la probabilité qu'un événement de décompensation d'insuffisance cardiaque d'un patient soit imminent, le circuit de traitement est configuré pour :
comparer chaque paramètre de la pluralité de paramètres physiologiques détectés à des seuils spécifiques aux paramètres ; et
appliquer un modèle probabiliste bayésien aux résultats des comparaisons, éventuellement, dans lequel la pluralité de paramètres physiologiques détectés comprend au moins deux éléments parmi : indice fluidique, activité du patient, charge tachycardie atriale (AT)/fibrillation atriale (FA), fréquence ventriculaire pendant AT/AF, pourcentage de stimulation ventriculaire, chocs, tachycardie ventriculaire/fibrillation ventriculaire traitées, fréquence ventriculaire nocturne, variabilité de fréquence cardiaque, sons cardiaques ou sons pulmonaires.
